(19) Europäisches Patentamt
European Patent Office
Office européen des brevets

(11) **EP 4 168 056 B1**

(12) **EUROPÄISCHE PATENTSCHRIFT**

(45) Veröffentlichungstag und Bekanntmachung des Hinweises auf die Patenterteilung:
**09.04.2025 Patentblatt 2025/15**

(21) Anmeldenummer: **21735141.0**

(22) Anmeldetag: **18.06.2021**

(51) Internationale Patentklassifikation (IPC):
**A61L 9/20** *(2006.01)* **C02F 1/32** *(2023.01)*
**F21V 7/00** *(2006.01)*

(52) Gemeinsame Patentklassifikation (CPC):
**A61L 9/20; C02F 1/325;** A61L 2209/14;
A61L 2209/15; C02F 1/001; C02F 2201/3222;
C02F 2201/3228

(86) Internationale Anmeldenummer:
**PCT/EP2021/066577**

(87) Internationale Veröffentlichungsnummer:
**WO 2021/255234 (23.12.2021 Gazette 2021/51)**

(54) **BESTRAHLUNGSVORRICHTUNG ZUR UV-BESTRAHLUNG EINES STRÖMENDEN MEDIUMS**

IRRADIATION DEVICE FOR UV IRRADIATION OF A FLOWING MEDIUM

DISPOSITIF DE RAYONNEMENT DESTINÉ AU RAYONNEMENT UV À UN MILIEU EN CIRCULATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2020 EP 20181097**

(43) Veröffentlichungstag der Anmeldung:
**26.04.2023 Patentblatt 2023/17**

(73) Patentinhaber: **Virobuster International GmbH 53578 Windhagen (DE)**

(72) Erfinder: **YIGIT, Fehmi 48599 Gronau (DE)**

(74) Vertreter: **Von Rohr Patentanwälte Partnerschaft mbB**
**Rüttenscheider Straße 62**
**45130 Essen (DE)**

(56) Entgegenhaltungen:
EP-A- 2 928 506       EP-A1- 3 111 963
WO-A1-93/07091       DE-A1- 102017 117 324
DE-U1- 20 021 236     US-A1- 2005 092 932
US-A1- 2019 049 129

- DATABASE WPI Week 201782, Derwent World Patents Index; AN 2017-79856P, XP002804396
- DATABASE WPI Week 201146, Derwent World Patents Index; AN 2011-E35055, XP002805061

**Beschreibung**

Die Erfindung betrifft eine Bestrahlungsvorrichtung zur UV-Bestrahlung, insbesondere UV-C-Bestrahlung, eines die Bestrahlungsvorrichtung durchströmenden Mediums, insbesondere Wasser, Luft und/oder ein Gasgemisch und/oder ein Dampfgemisch, insbesondere zur Inaktivierung und/oder Abtötung von in dem Medium befindlichen Mikroorganismen, wie Bakterien, Keime, Schimmel und/oder Viren. Die Bestrahlungsvorrichtung weist einen Einlass und einen Auslass für das Medium bzw. den Mediumstrom auf, wobei der Einlass und der Auslass an einem von dem Medium durchströmten Gehäuse vorgesehen sind. Im Inneren des Gehäuses ist eine UV-Strahlung, insbesondere UV-C-Strahlung, emittierende Strahlungsquelle zur Bestrahlung des das Gehäuses durchströmenden Mediums angeordnet.

Insbesondere betrifft die vorliegende Erfindung das technische Gebiet der sogenannten UV-Desinfektion. Der Begriff "UV-Desinfektion" bezeichnet Verfahren, bei denen Mikroorganismen - auch als Mikroben bezeichnet - durch die Behandlung mit UV-Strahlung abgetötet werden können. Dabei kann die UV-Desinfektion zur Trinkwasseraufbereitung, zur Oberflächendesinfizierung, zur Abluftaufbereitung aber auch im Bereich der hygienischen Verarbeitung von Lebensmitteln oder dergleichen eingesetzt werden. Auch Luft in öffentlichen Bereichen kann so "sauber" - das heißt eine geringe Belastung mit schädlichen Mikroorganismen aufweisend - gehalten werden.

Als UV-C-Strahlung wird nachfolgend eine Strahlung mit einer Wellenlänge zwischen 100 nm bis 280 nm verstanden, insbesondere wobei im Rahmen der UV-Desinfektion UV-C-Strahlung bei einer Wellenlänge zwischen 200 nm bis 280 nm, vorzugsweise 240 nm bis 280 nm, eingesetzt wird.

Die UV-Desinfektion nutzt insbesondere eine Wellenlänge der UV-Strahlung zwischen 200 bis 300 nm. Dabei wirkt die abgegebene UV-Strahlung bakterizid - das heißt sie wird von der DNA absorbiert und zerstört deren Struktur und inaktiviert lebende Zellen. So können Mikroorganismen, wie Viren, Bakterien, Hefen und Pilze, mit UV-Strahlung innerhalb kürzester Zeit, insbesondere innerhalb von wenigen Sekunden, unschädlich gemacht werden.

Bei ausreichend hoher Bestrahlungsstärke ist die UV-Desinfektion somit eine zuverlässige und ökologische Methode, da insbesondere keine Zugabe von weiteren Chemikalien notwendig ist. Besonders vorteilhaft ist, dass Mikroorganismen keine Resistenz gegen UV-Strahlung entwickeln können. Letztlich kann durch die UV-Desinfektion auch die Vermehrung der Mikroorganismen unterbrochen werden.

Das Verfahren der UV-Desinfektion kann auch als "Ultraviolet Germicidal Irritation" (UVGI) und/oder als Mikroben-Desinfektion bezeichnet werden, insbesondere wobei UV-Strahlung mit einer Wellenlänge von 254 nm eingesetzt wird. Der Einsatz der UV-Desinfektion ist zur Virusinaktivierung bei der Luftreinigung besonders vorteilhaft, da so ermöglicht wird, große Luftvolumina von Viren zu befreien.

Der Ausbruch der Corona-Pandemie Anfang 2020 zeigt die dringende Notwendigkeit, wirtschaftliche, effiziente und ökologische Verfahren zur Virusinaktivierung vorzuhalten.

Ziel von Weiterentwicklungen ist es, Vorrichtungen bereitzustellen, die es ermöglichen, Medienströme mit einem großen Durchsatz, insbesondere Luftströme, effizient reinigen zu können. Ein weiteres oder alternatives Ziel ist es, auch diejenigen Partikel abzutöten, die vergleichsweise resistent gegen UV-Strahlung sind. Letztlich sind verschiedene Grundstrahlungsdosen bekannt, die in Abhängigkeit der zu tötenden Mikroorganismen voneinander abweichen können.

Die UV-Strahlungsdosis ($J/m^2$) ergibt sich aus der Multiplikation der UV-Bestrahlungsintensität ($W/m^2$) und der UV-Bestrahlungszeit (s).

Letztlich hängt die Inaktivierung der Mikroorganismen insbesondere von der Leistung oder der erreichten Intensität der UV-Strahlung ab. Auch die Distanz des Mediumstromes zur Strahlungsquelle hat einen Einfluss auf die Effizienz der Desinfektion bzw. Reinigung.

Die US 2019/0491239 A1 betrifft eine UV-Röhre zur Abtötung von Mikroorganismen, die in einer Flüssigkeit enthalten sind, die durch ein damit verbundenes Flüssigkeitsantriebsmittel zugeführt wird.

Die DE 10 2017 117 324 A1 betrifft eine Desinfektionsanlage für Fluide, aufweisend einen mit dem zu desinfizierenden Fluid durchströmbaren Gehäusekörper mit einem Einlass und einem Auslass und mindestens einer UV-Lichtquelle zur Bestrahlung des durch den Gehäusekörper strömenden Fluids.

Die DE 200 21 236 U1 betrifft eine Vorrichtung zum Reinigen von Luft mit einer Bestrahlungslampe, die im Inneren eines einen Luftströmungsweg umschließenden Gehäuses untergebracht ist.

Die EP 3 111 963 A1 betrifft ein System zur Behandlung von Flüssigkeiten, insbesondere ein System zur Behandlung von Wasser mit ultravioletter Strahlung.

Die KR 2017 0124845 A bezieht sich auf einen Ultraviolett-Luftsterilisator und ein Ultraviolett-Luftsterilisationssystem.

Die WO 93/07091 A1 betrifft ein Abwasserdesinfektionssystem und insbesondere ein Durchflusssystem, das ultraviolette Strahlung zur Desinfektion von Abwasser verwendet.

Die RU 2 417 105 C1 bezieht sich auf Desinfektionsmittel und kann für die bakterientötende Luftdesinfektion durch ultraviolette Strahlung verwendet werden.

Die US 2005/092932 A1 bezieht sich auf eine Vorrichtung zur Behandlung von Fluiden mit ultraviolettem ("UV") Licht und stellt insbesondere eine Vorrichtung bereit, die die UV-Dosis gleichmäßig verteilt, um eine erhöhte Behandlungswirksamkeit zu erzielen.

Aufgabe der vorliegenden Erfindung ist es, eine verbesserte Bestrahlungsvorrichtung zur UV-Bestrahlung, insbesondere UV-C-Bestrahlung, bereitzustellen, die bevorzugt eine effizientere Inaktivierung der Mikroorganismen ermöglicht.

Die erfindungsgemäße Aufgabe wird zumindest im Wesentlichen durch eine Bestrahlungsvorrichtung nach Anspruch 1 gelöst.

Die Bestrahlungsvorrichtung ist zur UV-Bestrahlung, insbesondere UV-C-Bestrahlung, eines die Bestrahlungsvorrichtung durchströmenden, vorzugsweise gasförmigen und/oder fluiden, Mediums insbesondere Wasser oder Luft, ausgebildet, wobei die Bestrahlungsvorrichtung ein einen Einlass und einen Auslass für das Medium aufweisendes und zu durchströmendes Gehäuse aufweist. Das Gehäuse kann von dem Medium durchströmt werden. Die Bestrahlungsvorrichtung kann insbesondere zur Inaktivierung von in dem Medium befindlichen Mikroorganismen, wie Bakterien, Keime, Schimmel und/oder Viren, verwendet werden. Die Bestrahlungsvorrichtung umfasst weiter wenigstens eine im Inneren des Gehäuses angeordnete und UV-Strahlung, insbesondere UV-C-Strahlung, emittierende Strahlungsquelle zur Bestrahlung des das Gehäuse durchströmenden Mediums.

Das Gehäuse umfasst einen Reflektor, wobei der Reflektor an der der Strahlungsquelle zugewandten Innenseite zumindest bereichsweise, vorzugsweise vollflächig, reflektierend mit einem Reflexionsgrad für die von der Strahlungsquelle emittierte UV-Strahlung von wenigstens 0,6 ausgebildet ist. Insbesondere kann der Reflektor auch durch die Innenseite des Gehäuses gebildet werden. Die Innenseite des Reflektors bzw. des Gehäuses ist insbesondere derart glatt ausgebildet, dass zumindest im Wesentlichen eine direkte bzw. gerichtete Reflektion der eintreffenden Strahlung erfolgen kann.

Die Bestrahlungsvorrichtung weist eine Halteeinrichtung auf, mittels derer die wenigstens eine Strahlungsquelle gehalten und/oder fixiert ist bzw. gehalten und/oder fixiert werden kann. Die Halteeinrichtung ist mit dem Gehäuse, vorzugsweise lösbar, verbunden. Die Halteeinrichtung kann derart ausgebildet sein, dass die Mittelachse der wenigstens einen Strahlungsquelle einen Winkel zur Mittelachse des Reflektors einschließt.

Erfindungsgemäß wird unter der Mittelachse insbesondere die Längsachse des Reflektors bzw. des Gehäuses oder der Strahlungsquelle verstanden. Die Mittelachse liegt bzw. verläuft insbesondere in der jeweiligen Mitte des Körpers und/oder im Schwerpunkt des jeweiligen Körpers und bildet vorzugsweise die Symmetrieachse. Sofern der Körper nicht symmetrisch ausgebildet ist, bildet die Mittelachse des jeweiligen Körpers - das heißt des Reflektors, des Gehäuses und/oder der Strahlungsquelle - die annähernde Symmetrieachse des Körpers. Somit werden erfindungsgemäß auch Mittelachsen von solchen Körpern umfasst, die nicht symmetrisch sind.

Insbesondere verläuft die Mittelachse der Strahlungsquelle oder des Reflektors bzw. des Gehäuses durch den Schwerpunkt und/oder den Mittelpunkt der Strahlungsquelle bzw. des Gehäuses. Die Mittelachse verläuft vorzugsweise in Längserstreckung des Reflektors bzw. des Gehäuses oder der Strahlungsquelle, wobei die Strahlungsquelle oder der Reflektor bzw. das Gehäuse langgestreckt ausgebildet sind.

Eine Längserstreckung ist insbesondere derart zu verstehen, dass die Länge des Körpers die Breite des Körpers übersteigt.

Bei einer besonders bevorzugten Ausführungsform ist vorgesehen, dass der eingeschlossene Winkel zwischen der Mittelachse der wenigstens einen Strahlungsquelle und der Mittelachse des Reflektors zwischen $\arcsin((0,2 \cdot D) / L)$ und $\arcsin((4 \cdot D) / L)$ liegt. Somit kann insbesondere der Abstand zwischen dem einen stirnseitigen Ende der Strahlungsquelle und dem anderen stirnseitigen Ende bzw. die erzeugte Maximalverschiebung zwischen $0,2 \cdot D$ und $4 \cdot D$ liegen. In diesem Zusammenhang gibt D den, insbesondere maximalen, Durchmesser der Strahlungsquelle an, wobei L die Länge der Strahlungsquelle referenziert. Insgesamt wird also ein Schrägversatz über die gesamte Länge der jeweiligen Strahlungsquelle zwischen $0,2 \cdot D$ bis $4 \cdot D$ erreicht.

In diesem Zusammenhang versteht es sich, dass die Strahlungsquellen (zueinander) die gleiche oder auch eine unterschiedliche Länge oder Durchmesser aufweisen können. Das vorgenannte Verhältnis des Winkels bezieht sich dabei auf die jeweilige Länge und den jeweiligen Durchmesser der betrachteten Strahlungsquelle. Sofern der Durchmesser der Strahlungsquelle variiert, bezeichnet D insbesondere den maximalen und/oder den mittleren Durchmesser.

Besonders bevorzugt liegt der eingeschlossene Winkel zwischen der Mittelachse der wenigstens einen Strahlungsquelle und der Mittelachse des Reflektors zwischen $\arcsin((0,5 \cdot D) / L)$ und $\arcsin((3 \cdot D) / L)$, weiter bevorzugt zwischen $\arcsin(D / L)$ und $\arcsin((2 \cdot D) / L)$. Alternativ oder zusätzlich kann vorgesehen sein, dass der eingeschlossene Winkel zwischen der Mittelachse der wenigstens einen Strahlungsquelle und der Mittelachse des Reflektors zwischen 0,5° bis 15°, weiter bevorzugt zwischen 2° bis 10°, vorzugsweise zwischen 2° +/- 0,5°, liegt.

Ferner ist bei einer weiteren bevorzugten Ausführungsform vorgesehen, dass die Strahlungsquelle zumindest im Wesentlichen stabförmig ausgebildet ist. Alternativ oder zusätzlich kann vorgesehen sein, dass die Strahlungsquelle zumindest im Wesentlichen zylinderförmig und/oder langgestreckt ausgebildet ist. Insbesondere verläuft die Längserstreckung der Strahlungsquelle zumindest im Wesentlichen - das heißt unter Berücksichtigung der schrägen und/oder winkligen Anordnung der Strahlungsquelle im Reflektor - in Längsrichtung des Gehäuses bzw. des Reflektors. Demgemäß verläuft die Längserstreckung der Strahlungsquelle insbesondere nicht orthogonal zur Längserstreckung des Gehäuses und/oder des Reflektors.

Erfindungsgemäß, dass eine Mehrzahl von Strahlungsquellen an der Halteeinrichtung gehalten und/oder fixiert ist. Insbesondere schließt jede Mittelachse jeder Strahlungsquelle einen Winkel, bevorzugt in der vorgenannten Größenordnung, zu der Mittelachse des Reflektors ein.

Bei einer weiteren bevorzugten Ausführungsform sind die Mittelachsen, insbesondere wenigstens zwei Mittelachsen, weiter bevorzugt wenigstens vier Mittelachsen, insbesondere alle Mittelachsen, der Strahlungsquellen zueinander parallel angeordnet. Alternativ oder zusätzlich kann vorgesehen sein, dass wenigstens zwei Mittelachsen, bevorzugt wenigstens drei Mittelachsen, weiter bevorzugt wenigstens vier Mittelachsen, der Strahlungsquellen jeweils zueinander versetzt, vorzugsweise schräg, angeordnet sind. Demnach können die Strahlungsquellen auch miteinander verdreht, tordiert und/oder verdrillt angeordnet sein. Insbesondere kann der eingeschlossene Winkel zwischen zwei benachbarten Strahlungsquellen, insbesondere zwischen den benachbarten Mittelachsen der benachbarten Strahlungsquellen, zwischen 1° bis 120°, weiter bevorzugt zwischen 5° bis 90°, weiter bevorzugt zwischen 10° bis 40°, betragen. Der vorgenannte Winkel gibt insbesondere den Grad der Verdrillung zwischen den Strahlungsquellen an.

Besonders bevorzugt sind die Mittelachsen der Strahlungsquellen zueinander schräg und/oder windschief angeordnet.

Darüber hinaus ist bei einer weiteren bevorzugten Ausführungsform vorgesehen, dass die Strahlungsquelle lösbar mit der Halteeinrichtung verbunden ist. Bevorzugt ist jede Strahlungsquelle lösbar mit der Halteeinrichtung verbunden. Eine lösbare Anordnung ermöglicht den Vorteil, dass eine Strahlungsquelle, die beschädigt ist oder ausgetauscht werden muss, aus der Halteeinrichtung entnommen werden kann. **In** diesem Zusammenhang kann vorgesehen sein, dass die Strahlungsquelle gemeinsam mit der Halteeinrichtung zunächst aus dem Reflektor entfernt wird, wobei anschließend ein Austausch der Strahlungsquelle erfolgt.

Zudem ist vorzugsweise bei einer weiteren Ausgestaltung des Erfindungsgedankens vorgesehen, dass die Halteeinrichtung für wenigstens eine Strahlungsquelle ein Verstellmittel zur Verstellung der Schrägstellung der Mittelachse der Strahlungsquelle im Bezug zur Mittelachse des Reflektors aufweist. Das Verstellmittel kann insbesondere derart ausgebildet sein, dass es im gelösten Zustand eine Verstellung ermöglicht und anschließend durch eine Feststellung die Strahlungsquelle fest mit der Halteeinrichtung verbindet, so dass insbesondere keine Verstellung der Strahlungsquelle im festgestellten Zustand des Verstellmittels erfolgen kann. Das Verstellmittel kann beispielsweise eine Schraubverbindung umfassen. Auch kann das Verstellmittel eine Verschiebeeinrichtung aufweisen und/oder das Verstellmittel ist zumindest abschnittsweise teleskopierbar ausgebildet.

Darüber hinaus ist bei einer weiteren bevorzugten Ausführungsform vorgesehen, dass die Strahlungsquellen zueinander gleich beabstandet sind. Insbesondere erstreckt sich die Gleich-Beabstandung der Strahlungsquellen über die gesamte Länge der jeweiligen Strahlungsquellen. Alternativ oder zusätzlich kann vorgesehen sein, dass, wie zuvor erläutert, wenigstens zwei, insbesondere wenigstens zwei unmittelbar benachbarte, Strahlungsquellen mit ihrer Mittelachse einen unterschiedlichen Winkel zu der Mittelachse des Reflektors einschließen. Demnach können die Strahlungsquellen auch zueinander tordiert und/oder verdrillt angeordnet sein, was erfindungsgemäß die zuvor angesprochenen vorteilhaften Effekte der Erhöhung der konstruktiven Interferenz zur Folge hat.

Eine parallele Beabstandung der Strahlungsquellen ist mit dem Vorteil verbunden, dass eine einfache Handhabung des "Lampenpaketes" - das heißt der an der Halteeinrichtung befestigten Strahlungsquellen - erfolgen kann sowie ein einfaches Austauschen der einzelnen Strahlungsquellen, da nicht auf eine etwaige Verdrillung zwischen den Strahlungsquellen geachtet werden muss.

Erfindungsgemäß weist die Halteeinrichtung eine erste Halteeinheit auf. Die erste Halteeinheit ist über ein erstes Verbindungsmittel der Halteeinrichtung mit dem Gehäuse und/oder dem Reflektor lösbar verbindbar und/oder verbunden. Ferner weist die erste Halteeinrichtung eine Mehrzahl von zumindest bereichsweise zueinander beabstandeten ersten Haltemitteln auf. Die Haltemittel sind als, vorzugsweise stegförmige, Haltearme ausgebildet sein.

In diesem Zusammenhang versteht es sich, dass die Haltearme bzw. die ersten Haltemittel der ersten Halteeinheit zueinander unterschiedlich ausgebildet sein können. An den ersten Haltemitteln können die Strahlungsquellen derart befestigt werden, dass jeweils ein erstes Haltemittel einer Strahlungsquelle zugeordnet ist und zur Befestigung einer Strahlungsquelle dient.

Eine Beabstandung der Strahlungsquellen zueinander kann somit über eine zumindest bereichsweise vorgesehene Beabstandung der ersten Haltemittel erreicht werden. Dadurch, dass die ersten Haltemittel der ersten Halteeinheit zugeordnet und alle ersten Haltemittel miteinander zumindest mittelbar verbunden sind, kann eine kompakte Ausbildung der gesamten Strahlungseinheit - d.h. die die Strahlungsquellen aufweisende Strahlungseinheit - erreicht werden. Die Strahlungseinheit kann auch als Lampenpaket bezeichnet werden. Die erste Halteeinheit ermöglicht es daher, die Strahlungseinheit kompakt dem Gehäuse und/oder dem Reflektor zu entnehmen und so letztlich eine einfache Montage und Demontage der Strahlungseinheit - beispielsweise zu Wartungszwecken - aus dem Gehäuse zu ermöglichen. Auch stellt die erste Halteeinheit sicher, dass die Strahlungsquellen im Hinblick auf ihre Ausrichtung stets im Gehäuse und/oder im Reflektor fixiert angeordnet sind.

Durch die Beabstandung der ersten Haltemittel kann insbesondere eine zumindest im Wesentlichen sonnenförmige und/oder sternförmige Ausbildung der ersten Halteeinheit erreicht werden, wobei die ersten Haltemittel ausgehend von einem gemeinsamen Ausgangspunkt abstehen.

Ferner sind die ersten Haltemittel mit einem Verbindungsbereich der ersten Halteeinheit verbunden. Der Verbindungsbereich ist mit dem Verbindungsmittel, vorzugsweise unmittelbar, verbunden und/oder einstückig mit diesem ausgebildet. Letztlich können die ersten Haltemittel über den Verbindungsbereich mit dem Verbindungsmittel und somit lösbar mit dem Gehäuse verbunden werden. Die ersten Haltemittel können mit jeweils einem Endbereich mit dem Verbindungsbereich verbunden sein. Der weitere Endbereich des, vorzugsweise langgestreckten, ersten Haltemittels kann frei angeordnet sein - das heißt nicht mit einem Bestandteil der ersten Halteeinheit unmittelbar verbunden sein.

Letztlich kann somit das erste Haltemittel als Tragarm und/oder Kragarm ausgebildet sein, der an einem Endbereich an dem Verbindungsbereich angeordnet und/oder gelagert ist. Der Verbindungsbereich kann somit das Zentrum der ersten Halteeinheit bilden. Der Verbindungsbereich muss allerdings nicht im Schwerpunkt der ersten Halteeinheit angeordnet sein, sondern kann auch außerhalb des Schwerpunktes der ersten Halteeinheit angeordnet sein.

Zudem ist die Strahlungsquelle an einem stirnseitigen Endbereich mit dem ersten Haltemittel verbunden. Die Verbindung zu dem ersten Haltemittel kann insbesondere zum einen lösbar und zum anderen formschlüssig, kraftschlüssig und/oder reibschlüssig ausgebildet sein. **In** diesem Zusammenhang versteht es sich, dass nicht zwingend die Stirnseite der Strahlungsquelle mit dem ersten Haltemittel verbunden sein muss, sondern der stirnseitige Endbereich umfasst letztlich den Bereich der Strahlungsquelle, der die Stirnseite einschließt. Somit ist die Strahlungsquelle letztlich in wenigstens einem Endbereich an dem ersten Haltemittel fixiert und/oder mit diesem verbunden.

Das erste Haltemittel kann außerdem zur Befestigung mit der Strahlungsquelle wenigstens ein Befestigungsmittel, beispielsweise wenigstens einen Clip, wenigstens eine Klammer, wenigstens einen Federschenkel oder dergleichen aufweisen, das bzw. die zur lösbaren Anordnung der Strahlungsquelle ausgebildet sind/ist. Dieses Befestigungsmittel kann darüber hinaus vorzugsweise an dem ersten Haltemittel verschiebbar angeordnet sein, insbesondere so dass eine Justage der Anordnung der Strahlungsquellen erfolgen kann. Alternativ oder zusätzlich kann vorgesehen sein, dass das Befestigungsmittel einstückig mit den weiteren Bestandteilen des ersten Haltemittels ausgebildet ist. **In** diesem Zusammenhang versteht es sich, dass die Befestigungsmittel als Bestandteil des ersten Haltemittels angesehen werden.

Zudem kann erfindungsgemäß die Strahlungsquelle durch unterschiedliche Befestigungsarten mit dem ersten Haltemittel verbunden sein.

Des Weiteren kann vorgesehen sein, dass die Strahlungsquelle in dem Haltemittel zumindest bereichsweise aufgenommen ist, insbesondere so dass die Stirnseite gegenüber dem ersten Haltemittel absteht. Zur Aufnahme der Strahlungsquelle kann das erste Haltemittel ebenfalls entsprechende Befestigungsmittel, beispielsweise Spannklemmen, aufweisen.

Außerdem ist erfindungsgemäß vorgesehen, dass das erste Haltemittel, vorzugsweise alle ersten Haltemittel, mit dem einen Endbereich mit dem Verbindungsbereich verbunden ist. Zusätzlich kann vorgesehen sein, dass das erste Haltemittel an seinem freien Endbereich - insbesondere dem nicht-gelagerten Endbereich - mit der Strahlungsquelle verbunden ist. Auch in diesem Zusammenhang versteht es sich, dass der Endbereich des ersten Haltemittels nicht zwingend das äußerste Ende des ersten Haltemittels referenzieren muss, sondern der Endbereich kann das äußerste Ende und einen sich daran anschließenden Bereich umfassen. Der Endbereich des ersten Haltemittels erstreckt sich dabei über höchstens 30 %, vorzugsweise höchstens 20 %, der Länge des langgestreckten ersten Haltemittels.

Bei einer weiteren bevorzugten Ausführungsform ist vorgesehen, dass wenigstens ein erstes Haltemittel, bevorzugt wenigstens zwei erste Haltemittel, vorzugsweise alle ersten Haltemittel, über ein mit dem Verbindungsbereich verbundenes erstes Verstellmittel verstellbar ist. Das erste Verstellmittel kann beispielsweise eine Schraubverbindung, vorzugsweise in Kombination mit einem Langloch, und/oder eine teleskopierbare Verstellmöglichkeit oder dergleichen aufweisen.

Letztlich kann das erste Verstellmittel derart ausgebildet sein, dass die Schrägstellung der Mittelachse der an dem ersten Haltemittel befestigten Strahlungsquelle in Bezug zu der Mittelachse des Reflektors verstellbar ist. In diesem Zusammenhang versteht es sich, dass das erste Verstellmittel nur bedarfsweise "aktiviert" bzw. gelöst wird. So kann beispielsweise das erste Verstellmittel im gelösten Zustand eine Verstellung der Schrägstellung der Mittelachse der jeweiligen an dem ersten Haltemittel befestigten Strahlungsquelle ermöglichen, wobei nach erfolgter Ausrichtung eine erneute Feststellung bzw. Sicherung des ersten Verstellmittels erfolgen kann - beispielsweise durch ein Anziehen der Schraubverbindung. Dadurch kann im festgestellten Zustand des ersten Verstellmittels die Strahlungsquelle fest an dem ersten Haltemittel angeordnet sein, insbesondere so dass keine erneute Verstellung der Schrägstellung der Mittelachse der Strahlungsquelle möglich ist.

Wie zuvor erläutert, kann das erste Haltemittel langgestreckt ausgebildet sein. Alternativ oder zusätzlich kann vorgesehen sein, dass wenigstens zwei erste Haltemittel eine sich voneinander unterscheidende Länge aufweisen. Alternativ oder zusätzlich kann vorgesehen sein, dass das erste Haltemittel wenigstens zwei Anordnungsbereiche, vorzugsweise zur Anordnung der Befestigungsmittel, zur Verbindung mit der Strahlungsquelle aufweist.

Über die Anordnungsbereiche kann beispielsweise eine versetzte Anordnung der Strahlungsquellen in Bezug zueinander erreicht werden. So kann beispielsweise eine Strahlungsquelle an dem einen ersten Haltemittel weiter außen (in Bezug zum freien Endbereich des ersten Haltemittels) angeordnet sein als eine unmittelbar benachbarte Strahlungsquelle, die an einem weiteren ersten Haltemittel angeordnet ist, jedoch an einem gegenüber dem zuvor angesprochenen Anordnungsbereich versetzt angeordneten Anordnungsbereich.

Vorzugsweise ist die erste Halteeinheit derart ausgebildet, dass wenigstens zwei zwischen zwei unmittelbar benachbarten ersten Haltemitteln eingeschlossene Winkel zueinander unterschiedlich ausgebildet sind und insbesondere um wenigstens 5 %, bevorzugt wenigstens 10 %, zueinander abweichen bzw. sich voneinander unterscheiden. Besonders bevorzugt sind alle ersten Haltemittel zueinander derart angeordnet, dass alle eingeschlossenen Winkel zwischen unmittelbar benachbarten ersten Haltemitteln zueinander unterschiedlich sind. Dies kann zur weiteren vorteilhaften Wirkung beitragen, dass durch die schräge Anordnung der Strahlungsquellen in dem Reflektor konstruktive Interferenzeffekte erreicht werden können. Durch die unterschiedliche Anordnung der ersten Haltemittel, bevorzugt an dem ersten Verbindungsbereich, kann dieser Effekt nun weiter verstärkt werden.

Ferner kann die erste Halteeinheit zur Energiezuführung zu den Strahlungsquellen ausgebildet sein. Dabei können insbesondere in dem ersten Verbindungsmittel, in der ersten Halteeinheit und/oder in den ersten Haltemitteln Energieversorgungsleitungen, insbesondere Stromleitungen, Netzanschlussleitungen und dergleichen, angeordnet sein. Die Energieversorgungsleitungen können derart ausgebildet sein, dass die korrekte Funktionsweise der Strahlungsquellen, die vorzugsweise als LED ausgebildet sind, sichergestellt werden kann. Die zum Betrieb der Strahlungsquellen benötigte Energie kann somit den Strahlungsquellen über das erste Haltemittel und/oder über die erste Halteeinheit zugeführt werden. Somit können die im Inneren des Reflektors angeordneten Strahlungsquellen elektrisch bzw. energetisch angebunden werden.

In diesem Zusammenhang versteht es sich, dass etwaig vorgesehene Vorschaltgeräte, Netzanschlussteile und dergleichen außerhalb des Reflektors, insbesondere an der Außenseite des Gehäuses, angeordnet werden können und über Energieversorgungsleitungen, die durch die erste Halteeinheit geführt werden können, mit den Strahlungsquellen verbunden werden können. Demnach kann durch die vorteilhafte Leitungsführung vermieden werden, dass Energieversorgungsleitungen im Innenraum des Reflektors, insbesondere unbefestigt, mögliche destruktive Interferenz fördern könnten oder bei der Montage oder Demontage der Strahlungseinheit bzw. des Lampenpaketes stören oder gar zur Beschädigung des Lampenpaketes führen könnten.

Erfindungsgemäß ist vorgesehen, dass eine, vorzugsweise komplementär und/oder korrespondierend zu der ersten Halteeinheit ausgebildete, zweite Halteeinheit der Halteeinrichtung vorgesehen ist. Die zweite Halteeinheit kann zur Halterung bzw. Fixierung der Strahlungsquellen ausgebildet sein. Insbesondere sind die Strahlungsquellen mit dem weiteren stirnseitigen Endbereich mit der zweiten Halteeinheit zumindest mittelbar verbunden.

Die zweite Halteeinheit ist über wenigstens ein zweites Verbindungsmittel der Halteeinrichtung mit dem Gehäuse, vorzugsweise lösbar, verbindbar. Erfindungsgemäß weist die zweite Halteeinheit eine Mehrzahl von zueinander zumindest bereichsweise beabstandeten zweiten Haltemitteln, vorzugsweise ausgebildet als, insbesondere stegförmige, Haltearme, auf.

In diesem Zusammenhang versteht es sich, dass die zweiten Haltearme bzw. die zweiten Haltemittel der zweiten Halteeinheit zueinander unterschiedlich ausgebildet sein können. An den zweiten Haltemitteln können die Strahlungsquellen derart befestigt werden, dass jeweils ein zweites Haltemittel einer Strahlungsquelle zugeordnet ist und zur Befestigung einer Strahlungsquelle dient. Alternativ oder zusätzlich kann vorgesehen sein, dass jedem ersten Haltemittel ein korrespondierendes zweites Haltemittel zugordnet ist.

Eine Beabstandung der Strahlungsquellen zueinander kann somit über eine zumindest bereichsweise vorgesehene Beabstandung der zweiten Haltemittel erreicht werden. Dadurch, dass die zweiten Haltemittel der zweiten Halteeinheit zugeordnet und alle zweiten Haltemittel miteinander zumindest mittelbar verbunden sind, kann eine kompakte Ausbildung der gesamten Strahlungseinheit - d.h. die die Strahlungsquellen aufweisende Strahlungseinheit - erreicht werden. Die zweite Halteeinheit ermöglicht es daher gemeinsam mit der ersten Halteeinheit, die Strahlungseinheit kompakt dem Gehäuse und/oder dem Reflektor zu entnehmen und so letztlich eine einfache Montage und Demontage der Strahlungseinheit - beispielsweise zu Wartungszwecken - aus dem Gehäuse zu ermöglichen.

Durch die Beabstandung der zweiten Haltemittel kann insbesondere eine zumindest im Wesentlichen sonnenförmige und/oder sternförmige Ausbildung der zweiten Halteeinheit erreicht werden, wobei die zweiten Haltemittel ausgehend von einem gemeinsamen Ausgangspunkt abstehen.

Ferner können die zweiten Haltemittel mit einem zweiten Verbindungsbereich der zweiten Halteeinheit verbunden sein. Der zweite Verbindungsbereich kann mit dem zweiten Verbindungsmittel, vorzugsweise unmittelbar, verbunden sein und/oder einstückig mit diesem ausgebildet sein.

Letztlich können die zweiten Haltemittel über den zweiten Verbindungsbereich mit dem zweiten Verbindungsmittel und somit lösbar mit dem Gehäuse verbunden werden. Die zweiten Haltemittel können mit jeweils einem Endbereich mit dem zweiten Verbindungsbereich verbunden oder an diesem gelagert sein. Der weitere Endbereich des, vorzugsweise langgestreckten, zweiten Haltemittels kann frei angeordnet sein - das heißt nicht mit einem weiteren Bestandteil der zweiten Halteeinheit unmittelbar verbunden sein.

Ferner kann somit das zweite Haltemittel als Tragarm und/oder Kragarm ausgebildet sein, der an einem Endbereich an dem zweiten Verbindungsbereich angeordnet und/oder gelagert ist. Der zweite Verbindungsbereich kann somit das Zentrum der zweiten Halteeinheit bilden. Der zweite Verbindungsbereich muss allerdings nicht im Schwerpunkt der zweiten Halteeinheit angeordnet sein, sondern kann auch außerhalb des Schwerpunktes der zweiten Halteeinheit angeordnet sein.

Zudem ist die Strahlungsquelle an einem weiteren stirnseitigen Endbereich mit dem zweiten Haltemittel verbunden. Dabei kann die Strahlungsquelle insbesondere zwei stirnseitige Endbereiche aufweisen, die jeweils mit dem ersten bzw. zweiten Haltemittel verbunden sind.

Die Verbindung zu dem zweiten Haltemittel kann insbesondere zum einen lösbar und zum anderen formschlüssig, kraftschlüssig und/oder reibschlüssig ausgebildet sein. In diesem Zusammenhang versteht es sich, dass nicht zwingend die weitere Stirnseite der Strahlungsquelle mit dem zweiten Haltemittel verbunden sein muss, sondern der weitere stirnseitige Endbereich umfasst letztlich den Bereich der Strahlungsquelle, der die weitere Stirnseite einschließt.

Das zweite Haltemittel kann wie das erste Haltemittel außerdem zur Befestigung mit der Strahlungsquelle wenigstens ein Befestigungsmittel, beispielsweise wenigstens einen Clip, wenigstens eine Klammer, wenigstens einen Federschenkel oder dergleichen aufweisen, das bzw. die zur lösbaren Anordnung der Strahlungsquelle ausgebildet sind/ist. Dieses Befestigungsmittel kann darüber hinaus vorzugsweise an dem zweiten Haltemittel verschiebbar angeordnet sein, insbesondere so dass eine Justage der Anordnung der Strahlungsquellen erfolgen kann. Alternativ oder zusätzlich kann vorgesehen sein, dass das Befestigungsmittel einstückig mit den weiteren Bestandteilen des zweiten Haltemittels ausgebildet ist. In diesem Zusammenhang versteht es sich, dass die Befestigungsmittel als Bestandteil des zweiten Haltemittels angesehen werden.

Zudem kann erfindungsgemäß die Strahlungsquelle durch unterschiedliche Befestigungsarten mit dem zweiten Haltemittel verbunden sein. Bevorzugt sind die Befestigungsmittel der zweiten Haltemittel zumindest im Wesentlichen baugleich zu den Befestigungsmitteln des ersten Haltemittels ausgebildet.

Des Weiteren kann vorgesehen sein, dass die Strahlungsquelle in dem zweiten Haltemittel zumindest bereichsweise aufgenommen ist, insbesondere so dass die Stirnseite gegenüber dem zweiten Haltemittel absteht. Zur Aufnahme der Strahlungsquelle kann das zweite Haltemittel ebenfalls entsprechende Befestigungsmittel, beispielsweise Spannklemmen, aufweisen.

Außerdem ist bei einer weiteren bevorzugten Ausführungsform vorgesehen, dass das zweite Haltemittel, vorzugsweise alle zweiten Haltemittel, mit dem einen Endbereich mit dem zweiten Verbindungsbereich verbunden ist. Alternativ oder zusätzlich kann vorgesehen sein, dass das zweite Haltemittel an seinem freien Endbereich - insbesondere dem nicht-gelagerten Endbereich - mit der Strahlungsquelle verbunden ist. Auch in diesem Zusammenhang versteht es sich, dass der Endbereich des zweiten Haltemittels nicht zwingend das äußerste Ende des zweiten Haltemittels referenzieren muss, sondern der Endbereich kann das äußerste Ende und einen sich daran anschließenden Bereich umfassen. Der Endbereich des zweiten Haltemittels erstreckt sich dabei über höchstens 30 %, vorzugsweise höchstens 20 %, der Länge des langgestreckten zweiten Haltemittels.

Bei einer weiteren bevorzugten Ausführungsform ist vorgesehen, dass wenigstens ein zweites Haltemittel, bevorzugt wenigstens zwei zweite Haltemittel, vorzugsweise alle zweiten Haltemittel, über jeweils ein mit dem zweiten Verbindungsbereich verbundenes zweites Verstellmittel verstellbar ist. Das zweite Verstellmittel kann beispielsweise eine Schraubverbindung, vorzugsweise in Kombination mit einem Langloch, und/oder eine teleskopierbare Verstellmöglichkeit oder dergleichen aufweisen.

Letztlich kann das zweite Verstellmittel derart ausgebildet sein, dass die Schrägstellung der Mittelachse der an dem ersten Haltemittel befestigten Strahlungsquelle in Bezug zu der Mittelachse des Reflektors verstellbar ist. In diesem Zusammenhang versteht es sich, dass das zweite Verstellmittel nur bedarfsweise "aktiviert" bzw. gelöst wird. So kann beispielsweise das zweite Verstellmittel im gelösten Zustand eine Verstellung der Schrägstellung der Mittelachse der jeweiligen an dem zweiten Haltemittel befestigten Strahlungsquelle ermöglichen, wobei nach erfolgter Ausrichtung eine erneute Feststellung bzw. Sicherung des zweiten Verstellmittels erfolgen kann - beispielsweise durch ein Anziehen der Schraubverbindung. Dadurch kann im festgestellten Zustand des zweiten Verstellmittels die Strahlungsquelle fest an dem zweiten Haltemittel angeordnet sein, insbesondere so dass keine erneute Verstellung der Schrägstellung der Mittelachse der Strahlungsquelle gegeben ist.

Wie zuvor erläutert, kann das zweite Haltemittel langgestreckt ausgebildet sein. Alternativ oder zusätzlich kann vorgesehen sein, dass wenigstens zwei zweite Haltemittel eine sich voneinander unterscheidende Länge aufweisen. Alternativ oder zusätzlich kann vorgesehen sein, dass das zweite Haltemittel wenigstens zwei zweite Anordnungsbereiche, vorzugsweise zur Anordnung der Befestigungsmittel, zur Verbindung mit der Strahlungsquelle aufweist.

Vorzugsweise ist die zweite Halteeinheit derart ausgebildet, dass wenigstens zwei zwischen zwei unmittelbar benachbarten zweiten Haltemitteln eingeschlossene Winkel zueinander unterschiedlich ausgebildet sind und insbesondere um wenigstens 5 %, bevorzugt wenigstens 10 %, zueinander abweichen bzw. sich voneinander unterscheiden. Besonders bevorzugt sind alle zweiten Haltemittel zueinander derart angeordnet, dass alle eingeschlossenen Winkel zwischen unmittelbar benachbarten zweiten Haltemitteln zueinander unterschiedlich sind. Dies kann zur weiteren vorteilhaften Wirkung beitragen, dass durch die schräge Anordnung der Strahlungsquellen in dem Reflektor konstruktive Interferenzeffekte erreicht werden können. Durch die unterschiedliche Anordnung der zweiten Haltemittel, bevorzugt an dem zweiten Verbindungsbereich, kann dieser Effekt nun weiter verstärkt werden.

Vorzugsweise ist die erste Halteeinheit, insbesondere der Verbindungsbereich der ersten Halteeinheit, mit der zweiten Halteeinheit, insbesondere mit dem zweiten Verbindungsbereich, über ein langgestrecktes Verbindungteil verbunden. Das Verbindungteil kann insbesondere steif und/oder stabil ausgebildet sein. Bevorzugt ist das Verbindungteil an seiner dem Reflektor zugewandten Außenseite reflektierend, vorzugsweise mit einem Reflexionsgrad von wenigstens 0,6, bevorzugt von wenigstens 0,8, ausgebildet.

Das Verbindungteil kann die Stabilität der gesamten Strahlungseinheit bzw. des Lampenpaketes gewährleisten. So ermöglicht das Verbindungteil, dass die erste Halteeinheit und die zweite Halteeinheit nicht nur über die Strahlungsquellen miteinander verbunden sind, sondern auch über das Verbindungteil. Das Verbindungteil kann grundsätzlich unterschiedlich ausgebildet sein, beispielsweise zylinderförmig, rohrförmig oder dergleichen.

Insbesondere kann das Verbindungteil mittig zwischen den Strahlungsquellen angeordnet und/oder von den Strahlungsquellen umgeben sein. Somit kann das Verbindungteil im mittleren Bereich bzw. im Zentrum der Strahlungseinheit bzw. des Lampenpaketes angeordnet sein.

Weiter insbesondere ist das Verbindungteil nicht unmittelbar mit den ersten und/oder zweiten Haltemitteln und/oder mit den jeweiligen Strahlungsquellen verbunden. Vorzugsweise ist das Verbindungteil an den Verbindungsbereichen der ersten und zweiten Halteeinheit angeordnet. Das Verbindungteil ermöglicht somit ebenfalls eine einfache Montage und Demontage des gesamten Lampenpaketes und darüber hinaus auch eine Fixierung der Schrägstellung der Strahlungsquellen, insbesondere bei Durchströmung des Reflektors. So kann beispielsweise vermieden werden, dass die Strahlungsquellen im Hinblick auf ihre Ausrichtung im Verhältnis zur Mittelachse des Reflektors "wackeln" - und zwar aufgrund der an der Strahlungsquelle angreifenden Strömung des Mediums. Somit trägt das Verbindungteil nicht nur zur Stabilität, sondern auch zur verbesserten Funktionsweise der gesamten Bestrahlungsvorrichtung bei.

Besonders bevorzugt ist, dass die zweite Halteeinheit zumindest im Wesentlichen baugleich zur ersten Halteeinheit ausgebildet ist. So kann die zweite Halteeinheit insbesondere komplementär und/oder gespiegelt zur ersten Halteeinheit ausgebildet sein. Darüber hinaus kann die zweite Halteeinheit derart ausgebildet sein, dass die Schrägstellung und/oder die Verdrillung und/oder Tordierung der Strahlungsquellen zueinander sichergestellt werden kann.

Insbesondere ist vorgesehen, dass über die zweite Halteinheit keine Strom-und/oder Energiezuführung zu den Strahlungsquellen erfolgt. Die Energie- bzw. Stromversorgung der Strahlungsquellen kann insbesondere über in der ersten Halteeinheit vorgesehene Energieversorgungsleitungen gewährleistet werden. Die zweite Halteeinheit dient letztlich zur Fixierung und/oder Sicherung bzw. zur stabilen Ausrichtung der Strahlungsquellen in dem Reflektor.

Die zweite Halteeinheit kann ferner an einem anderen Bereich, beispielsweise an der gegenüberliegenden Seite des Reflektors und/oder des Gehäuses, angeordnet sein.

Vorzugsweise ist die erste Halteeinheit und/oder das erste Verbindungsmittel mit einem ersten Verbindungsabschnitt verbunden. Der erste Verbindungsabschnitt kann mit dem Gehäuse und/oder mit dem Reflektor lösbar verbindbar sein. Insbesondere steht der erste Verbindungsabschnitt zumindest teilweise über die Außenseite des Gehäuses ab bzw. erstreckt sich entlang der Außenseite des Gehäuses. Der erste Verbindungsabschnitt ermöglicht somit die Verbindung der ersten Halteeinheit nach außen, insbesondre zu der Außenseite des Gehäuses, die der Innenseite des Reflektors abgewandt ist.

Ferner kann an dem ersten Verbindungsabschnitt außenseitig eine erste Versorgungseinrichtung, vorzugsweise aufweisend eine Mehrzahl von Vorschaltgeräten, insbesondere zum Betrieb der Strahlungsquellen, anordnenbar und/oder verbindbar sein. Somit kann der erste Verbindungsabschnitt auch als "Andockbereich" für Netzgeräte, Vorschaltgeräte und dergleichen dienen, die beispielsweise in einer Versorgungseinrichtung angeordnet sind. Die erste Versorgungseinrichtung kann ferner elektrisch über den ersten Verbindungsabschnitt mit der ersten Halteeinheit verbunden sein.

Darüber hinaus kann das Haltesystem bzw. die Halteeinrichtung auch modular durch verschiedene Verbindungsabschnitte ausgebildet sein, die insbesondere unmittelbar aneinander angeordnet und vorzugsweise miteinander fest und lösbar verbindbar sind. So kann beispielsweise die zweite Halteeinheit und/oder das zweite Verbindungsmittel mit einem zweiten Verbindungsabschnitt verbunden sein. Auch der zweite Verbindungsabschnitt kann mit dem Gehäuse und/oder dem Reflektor lösbar verbindbar sein. Vorzugsweise steht auch der zweite Verbindungsabschnitt gegenüber der Außenseite des Gehäuses ab. Der zweite Verbindungsabschnitt kann mit dem ersten Verbindungsabschnitt mittelbar oder unmittelbar verbunden sein.

Bei einer mittelbaren Verbindung kann vorgesehen sein, dass wenigstens ein weiterer Verbindungsabschnitt vorgesehen ist, der lösbar mit dem ersten und/oder zweiten Verbindungsabschnitt formschlüssig und/oder reibschlüssig und/oder kraftschlüssig verbindbar und/oder verbunden ist. Dies ermöglicht letztlich den modularen Aufbau der Halteeinrichtung und darüber hinaus auch eine vergleichsweise einfache Variation der Länge der Halteinrichtung, die insbesondere zur Länge der verwendeten Strahlungsquelle korrelieren kann.

Besonders bevorzugt ist, dass der erste und der zweite Verbindungsabschnitt derart ausgebildet sind, dass sie lösbar miteinander formschlüssig und/oder reibschlüssig und/oder kraftschlüssig verbindbar und/oder verbunden sind. Bei einer solchen Verbindung könnten der erste und der zweite Verbindungsabschnitt unmittelbar miteinander verbunden werden.

Zur Verbindung der Verbindungsabschnitte können Verriegelungsmittel vorgesehen sein.

Insbesondere ragt der erste, zweite und/oder weitere Verbindungsabschnitt zumindest teilweise in das Innere des Reflektors und/oder grenzt an die Innenseite des Reflektors an. Der erste, zweite und/oder weitere Verbindungsabschnitt kann auch gegenüber der Innenseite des Reflektors zurückversetzt sein, der Innenseite des Reflektors jedoch, vorzugsweise unmittelbar, zugewandt sein. Vorzugsweise weisen der erste, zweite und/oder weitere Verbindungsabschnitt an der dem Innenraum des Reflektors zugewandten Außenfläche eine reflektierende Oberfläche auf, die vorzugsweise eine gerichtete bzw. direkte Reflektion mit einem Reflexionsgrad von wenigstens 0,6, bevorzugt wenigstens 0,8, weiter bevorzugt wenigstens 0,9, aufweist.

Der Reflektor kann beispielsweise derart ausgebildet sein, dass er an einem Profil, insbesondere einem Alu-Profil, montierbar ist. So kann beispielsweise der Reflektor als Blech, vorzugsweise Aluminium-Blech, ausgebildet sein, wobei die Form des Reflektors durch Einspannen des Reflektors an dem entsprechenden Profil und/oder den Verbindungsabschnitten ermöglicht werden kann. Die Verbindungsabschnitte können bei einer solchen Ausbildung des Reflektors für die Stabilität der gesamten Bestrahlungsvorrichtung sorgen bzw. diese Stabilität sicherstellen.

Besonders bevorzugt sind zwischen 3 bis 25, vorzugsweise zwischen 4 bis 15, weiter bevorzugt zwischen 5 bis 10, Strahlungsquellen, erste Haltemittel und/oder zweite Haltemittel vorgesehen.

Die Anzahl der Haltemittel ist insbesondere in Abhängigkeit der Anzahl der Strahlungsquellen ausgebildet. In diesem Zusammenhang kann vorgesehen sein, dass ein "Überhang" an ersten oder zweiten Haltemitteln vorhanden ist, so dass beispielsweise nicht an jedem Haltemittel eine Strahlungsquelle angeordnet sein muss.

Ferner kann die Anzahl der Strahlungsquellen in Abhängigkeit der gewünschten UV-Strahlungsdosis, der Länge des Reflektors, des zu behandelnden Volumenstroms des Mediums und dergleichen ausgebildet werden. Durch den insgesamt ermöglichten modularen Aufbau der gesamten Bestrahlungsvorrichtung kann auch eine individuelle Anpassung der Bestrahlung erfolgen. Beispielsweise kann auch eine Hinzufügung von Strahlungsquellen bedarfsweise ermöglicht werden, indem beispielsweise an dem jeweiligen Verbindungsbereich der ersten und/oder zweiten Halteeinheit weitere Haltemittel angeordnet werden oder bereits vorhandene "überschüssige" Haltemittel zur Montage der Strahlungsquellen genutzt werden.

Vorzugsweise sind wenigstens zwei, bevorzugt wenigstens drei, insbesondere alle, Strahlungsquellen zueinander baugleich ausgebildet. Hierdurch wird ein verbessertes Strahlungsbild erreicht, da die Strahlungsquellen zumindest im Wesentlichen die gleiche Strahlung aussenden.

Insbesondere weist wenigstens eine, bevorzugt alle, Strahlungsquellen einen Durchmesser D zwischen 1 cm bis 20 cm, bevorzugt zwischen 2 cm bis 10 cm, weiter bevorzugt zwischen 4 cm bis 6 cm, auf. Der vorgenannte Durchmesser kann der mittlere und/oder der maximale Durchmesser der Strahlungsquelle sein. Insbesondere ist vorgesehen, dass der Durchmesser der Strahlungsquelle zumindest im Wesentlichen konstant ist.

Vorzugsweise weist wenigstens eine, bevorzugt alle, Strahlungsquellen eine Länge zwischen 0,2 m bis 10 m, bevorzugt zwischen 0,5 m bis 5 m, weiter bevorzugt zwischen 1 m bis 2 m, auf.

Darüber hinaus kann der innere Durchmesser des Reflektors, insbesondere der innere maximale Durchmesser des Reflektors, zwischen 100 bis 1000 cm, bevorzugt zwischen 200 bis 600 cm, betragen. Insbesondere ist bei einem inneren Durchmesser von 250 cm +/- 20 % vorgesehen, dass fünf Strahlungsquellen eingesetzt werden. Je größer der innere Durchmesser des Reflektors ausgebildet ist, desto mehr Strahlungsquellen können eingesetzt werden. So kann beispielsweise vorgesehen sein, dass bei einem inneren Durchmesser des Reflektors von 350 cm +/-20 % in etwa sieben Strahlungsquellen eingesetzt werden. Bei einem inneren Durchmesser des Reflektors von 500 cm +/- 20 % können beispielsweise zehn Strahlungsquellen eingesetzt werden. In diesem Zusammenhang ist vorgesehen, dass der innere Durchmesser des Reflektors insbesondere zumindest im Wesentlichen konstant ausgebildet ist - und zwar über die Längserstreckung des Reflektors.

Besonders bevorzugt ist, wenn eine Auswerteeinrichtung zur Erfassung wenigstens einer chemischen und/oder physikalischen Größe vorgesehen ist. Die Auswerteeinrichtung kann beispielsweise in dem ersten Verbindungsabschnitt und/oder in der ersten Halteeinheit und/oder in der zweiten Halteeinheit und/oder im ersten und/oder zweiten Verbindungsmittel angeordnet sein. Insbesondere kann die Auswerteeinrichtung wenigstens einen Temperatursensor, UV-Sensor und/oder Geschwindigkeitssensor aufweisen. Die Auswerteeinrichtung kann zur Verbesserung der Funktionsweise der Bestrahlung dienen. So können die durch die Auswerteeinrichtung erfassten chemischen und/oder physikalischen Größen zur Steuerung und/oder Regelung der Bestrahlung, des Volumenstroms des Mediums und dergleichen dienen, so dass insbesondere eine optimierte Betriebsweise bereitgestellt werden kann.

Die Länge des ersten Haltemittels und/oder des zweiten Haltemittels kann zwischen $0,5 \cdot D_R$ bis $0,99 \cdot D_R$, bevorzugt zwischen $0,1 \cdot D_R$ bis $0,5 \cdot D_R$ betragen. In diesem Zusammenhang gibt $D_R$ den inneren, insbesondere maximalen, Durchmesser des Reflektors an. Somit können die ersten und/oder zweiten Haltemittel sowohl mittig im Reflektor, als auch außerzentrisch im Reflektor angeordnet sein. Die Ausbildung der Länge der Haltemittel kann darüber hinaus auch in der jeweiligen Halteeinheit - das heißt in der ersten und/oder zweiten Halteeinheit - variieren.

Besonders bevorzugt ist wenigstens eine, weiter bevorzugt wenigstens zwei, insbesondere alle, Strahlungsquellen als LED ausgebildet. LEDs haben sich bei der Bestrahlung zur Abtötung von Mikroorganismen im Medium als besonders vorteilhaft herausgestellt, insbesondere da sie sich durch eine Langlebigkeit auszeichnen.

Das Innere des Gehäuses bzw. der umschlossene Innenraum des Gehäuses kann insbesondere als UV-Behandlungskammer zur Behandlung des Mediums angesehen werden.

Bevorzugt ist die Innenwandung ein Reflektor, der weiter bevorzugt als in das Gehäuse eingeschobene und/oder austauschbare Gehäusekomponente ausgebildet ist.

Unter dem Reflexionsgrad wird im Sinne der Erfindung das Verhältnis zwischen reflektierter und einfallender Intensität als Energiegröße verstanden. Der Reflexionsgrad kann insbesondere in Abhängigkeit des Materials der Innenwandung, auf die die Strahlung auftrifft, und von der Strahlung selbst abhängen.

Die Längsachse des Gehäuses oder der Strahlungsquelle bezeichnet dabei insbesondere diejenige Achse, die in Richtung der längeren bzw. größten Ausdehnung des Körpers verläuft und somit die in Längsrichtung verlaufende Achse darstellt. Die Längsachse kann - muss jedoch nicht - eine annähernde Symmetrieachse des Gehäuses oder Strahlungsquelle sein.

Erfindungsgemäß gelingt es insbesondere, auch Bakterien, Viren und/oder Pilze mit einer vergleichsweise hohen UV-Resistenz abzutöten.

Die Abtötungsrate der Mikroorganismen kann insbesondere nach folgender Formel bestimmt werden:

$$S(t) = S_o\, e^{-I \cdot k \cdot t}$$

mit:

I = Intensität ($W/m^2$),
k = UV-Rate des Mikroorganismus ($m^2/J$),
t = die Verweilzeit (s)
S = Mikrobengehalt
$S_o$ = Anfangsgehalt der Mikroben.

Die Innenwandung des Gehäuses und/oder die Innenseite des Gehäuses kann mit einer UV-Strahlung reflektierenden Beschichtung, die insbesondere den gewünschten Reflexionsgrad der Innenseite des Gehäuses sicherstellt, zumindest bereichsweise, vorzugsweise vollflächig bzw. vollständig, beschichtet sein. Alternativ oder zusätzlich kann vorgesehen sein, dass die Innenwandung und/oder die Innenseite des Gehäuses als Material Metall aufweist und/oder daraus besteht. Insbesondere kann die Innenwandung ein Blech, insbesondere ein Aluminium aufweisendes Blech und/oder ein verzinktes Blech und/oder ein Edelstahlblech, aufweisen und/oder daraus bestehen. Dabei kann - wie zuvor erläutert - die Innenwandung des Gehäuses als in das Gehäuse einsetzbare Komponente ausgebildet sein. Besonders bevorzugt ist ein mehrmals beschichtetes Aluminiumblech als Material für die Innenwandung des Gehäuses vorgesehen.

Darüber hinaus ist bei einer weiteren bevorzugten Ausführungsform vorgesehen, dass die Innenwandung und/oder die Innenseite des Gehäuses zylinderförmig und/oder gewellt oder eben - insbesondere flach bzw. ohne Erhebungen - ausgebildet ist. Alternativ oder zusätzlich kann vorgesehen sein, dass die Innenwandung und/oder die Innenseite des Gehäuses eine Mehrzahl von Erhebungen und/oder Vertiefungen aufweist und/oder dass die Innenwandung des Gehäuses rotationssymmetrisch ausgebildet ist. In weiteren Ausführungsformen kann die Innenwandung des Gehäuses auch nicht rotationssymmetrisch ausgebildet sein.

Bei einer weiteren bevorzugten Ausführungsform ist die Längsachse des Gehäuses, insbesondere die Längsachse des Innenraums bzw. der Behandlungskammer des Gehäuses und/oder der Innenwandung des Gehäuses, nicht koaxial zur Längsachse der Strahlungsquelle ausgerichtet. Besonders bevorzugt ist die Strahlungsquelle insbesondere nicht symmetrisch im Innenraum des Gehäuses angeordnet.

Bei einer weiteren ganz besonders bevorzugten Ausführungsform ist die Längsachse des Gehäuses, insbesondere die Längsachse des Innenraums bzw. der Behandlungskammer des Gehäuses und/oder der Innenwandung des Gehäuses, versetzt, vorzugsweise schräg, zu der Längsachse der Strahlungsquelle ausgerichtet. Insbesondere ist die Längsachse des Gehäuses, insbesondere des Innenraums des Gehäuses und/oder der Innenwandung des Gehäuses, mit einem Winkel $\alpha$ größer als 2°, bevorzugt zwischen 2° bis 50°, weiter bevorzugt zwischen 3° bis 15°, zu der Längsachse der Strahlungsquelle ausgerichtet bzw. schließt einen Winkel α in der vorgenannten Größenordnung zu der Längsachse der Strahlungsquelle ein.

Vorzugsweise ist die Strahlungsquelle dezentral - insbesondere nicht-mittig - in dem und/oder mit Bezug zu dem Gehäuse angeordnet. Durch die dezentrale Anordnung können die konstruktiven Interferenzeffekte hervorgerufen werden, da eine Positionierung der Strahlungsquelle insbesondere derart vorgenommen wird, dass die, vorzugsweise gerichtete, reflektierte Strahlung mit der von der Strahlungsquelle emittierten Strahlung konstruktiv interferiert.

Vorzugsweise weist das Gehäuse eine Länge zwischen 30 bis 200 cm, bevorzugt zwischen 80 bis 150 cm, auf. Das Gehäuse kann einen Durchmesser, vorzugsweise einen Innendurchmesser, zwischen 8 bis 30 cm, bevorzugt zwischen 10 bis 20 cm, aufweisen. Bei einer besonders bevorzugten Ausführungsform weist das Gehäuse eine Länge zwischen 80 bis 150 cm und einen Innendurchmesser zwischen 10 bis 20 cm auf. Bei den vorgenannten Größenordnungen kann insbesondere für einen Mediumstrom mit einer Strömungsgeschwindigkeit zwischen 1 bis 2 m/s eine effiziente Inaktivierung der Mikroorganismen gewährleistet werden.

Darüber hinaus kann die erfindungsgemäße Bestrahlungseinrichtung auch in Klimaanlagen oder Bürogebäuden oder dergleichen eingesetzt werden. Dabei versteht es sich, dass die Bestrahlungsvorrichtung in Abhängigkeit des Mediumstromes und/oder des Durchsatzes des Mediumstromes ausgebildet sein kann.

Ferner kann bei einer weiteren bevorzugten Ausführungsform vorgesehen sein, dass die Strahlungsquelle eine Mehrzahl von Leuchtmitteln, vorzugsweise LEDs, aufweist. Darüber hinaus kann alternativ oder zusätzlich die Strahlungsquelle eine zumindest im Wesentlichen langgestreckte und/oder stabförmige Form aufweisen. Insbesondere kann die Strahlungsquelle eine Länge von wenigstens 5 cm, bevorzugt zwischen 5 cm bis 30 cm, weiter bevorzugt zwischen 10 cm bis 20 cm, aufweisen. Bei einer besonders bevorzugten Ausführungsform ist vorgesehen, dass die Strahlungsquelle eine langgestreckten Form mit einer Mehrzahl von Leuchtmitteln, vorzugsweise LEDs aufweist, die entlang eines "arrays" angeordnet sind.

Zudem kann die Strahlungsquelle einen Durchmesser von wenigstens 1 cm, bevorzugt zwischen 1 cm und 20 cm, weiter bevorzugt zwischen 2 cm und 10 cm und insbesondere von 5 cm +/- 1 cm, aufweisen. Der Durchmesser der Strahlungsquelle kann insbesondere auch von dem Durchmesser des in der Strahlungsquelle eingesetzten Leuchtmittels abhängen.

Alternativ oder zusätzlich kann die Strahlungsquelle auch als UV-Niederdrucklampen, insbesondere eine Niederdruck-Quecksilber-Entladungslampe, und/oder als UV-Mitteldrucklampe ausgebildet sein.

Letztlich kann die Strahlungsquelle die zur Inaktivierung der Mikroorganismen benötigte UV-Strahlung, insbesondere die UV-C-Strahlung, bereitstellen. Dabei kann die Strahlungsquelle an ihrer Oberfläche eine Intensität zwischen 1000 bis 8000 W/m$^2$, bevorzugt zwischen 2000 bis 6000 W/m$^2$ und insbesondere von 4200 W/m$^2$ +/- 20 %, aufweisen.

Des Weiteren kann die Strahlungsquelle insbesondere eine Leistung von wenigstens 100 W, bevorzugt von 190 W +/- 10 %, erbringen. Die Leistung im Bereich der UV-C-Strahlung kann bevorzugt zwischen 10 bis 100 W, weiter bevorzugt zwischen 50 bis 70 W, betragen. Die von der Strahlungsquelle emittierte Strahlung kann mit ihrer Intensität im Abstandsquadrat abnehmen. Durch die erreichte konstruktive **In**terferenz kann dieser Reduzierung der Amplitude entgegengewirkt werden.

Bei einer weiteren bevorzugten Ausführungsform des Erfindungsgedankens ist vorgesehen, dass eine Mehrzahl von Strahlungsquellen in dem Gehäuse angeordnet ist. Insbesondere können in dem Gehäuse zwischen 2 bis 10, bevorzugt zwischen 2 bis 5, Strahlungsquellen angeordnet sein. Die Strahlungsquellen können jeweils insbesondere ebenfalls eine Mehrzahl von Leuchtmitteln, vorzugsweise LEDs, aufweisen. Durch die mehreren Strahlungsquellen kann über die Länge des Gehäuses eine zumindest im Wesentlichen gleichmäßige und/oder zur Inaktivierung der Mikroorganismen ausreichende Strahlung bereitgestellt werden.

Die Strahlung der mehreren Strahlungsquellen kann des Weiteren auch in dem entstehenden Interferenzbild in der UV-Behandlungskammer miteinander wechselwirken und zur Erhöhung der konstruktiven Interferenz beitragen. Die erfindungsgemäß angegebene Eignung zur konstruktiven Interferenz gilt insbesondere für jede Strahlungsquelle, die in der erfindungsgemäßen Bestrahlungsvorrichtung eingesetzt wird.

Grundsätzlich ist es auch möglich, dass die Strahlungsquellen voneinander unterschiedlich ausgebildet sind. Bevorzugt wird eine zumindest im Wesentlichen baugleiche Ausbildung der Strahlungsquellen vorgesehen.

Vorzugsweise sind die mehreren Strahlungsquellen zumindest bereichsweise überlappend in dem Gehäuse angeordnet. Die Länge des Überlappungsbereiches von wenigstens zwei Strahlungsquellen kann wenigstens 20 %, bevorzugt wenigstens 30 %, weiter bevorzugt zwischen 30 % bis 80 %, der Länge wenigstens einer Strahlungsquelle entsprechen. Der Überlappungsbereich kann insbesondere in Abhängigkeit der Länge des Gehäuses und/oder der Breite des Gehäuses und/oder in Abhängigkeit der unterschiedlichen Strahlungsquellen gewählt werden. Zudem ist beim Zustandekommen der Erfindung festgestellt worden, dass der Überlappungsbereich der Strahlungsquellen insbesondere nicht die erfindungsgemäße konstruktive Interferenz der Strahlung besonders nachteilig beeinflusst. So kann letztlich die Leistung der UV-Strahlung innerhalb des Gehäuses durch die mehreren Strahlungsquellen erhöht werden. Dies trägt ebenfalls zu einer effizienteren bzw. verbesserten Abtötung der Mikroben bei.

Bevorzugt können die mehreren Strahlungsquellen, insbesondere wenigstens zwei Strahlungsquellen, zueinander windschief und/oder schräg angeordnet sein. **In** einer alternativen oder zusätzlichen Ausführungsform kann auch vorgesehen sein, dass die Längsachsen der Strahlungsquellen zumindest im Wesentlichen parallel zueinander ausgerichtet sind.

Besonders bevorzugt sind die Längsachsen der Strahlungsquellen zueinander versetzt, vorzugsweise schräg, angeordnet. Die Längsachsen der Strahlungsquellen können einen Winkel β zwischen 1° bis 90°, bevorzugt zwischen 2° bis 50°, weiter bevorzugt zwischen 10° bis 30°, zueinander einschließen. Der Winkel β kann insbesondere in Abhängigkeit des hervorgerufenen Interferenzbildes innerhalb des Gehäuses gewählt werden, wobei im Interferenzbild eine möglichst hoher Anteil der konstruktiven Interferenz erreicht werden kann.

Bei einer weiteren bevorzugten Ausgestaltung ist vorgesehen, dass die Vorrichtung einen Ventilator zum Erzeugen des Mediumstromes vom Einlass zum Auslass umfasst. Der Ventilator ist insbesondere derart eingestellt, dass der Mediumstrom eine Durchströmungsgeschwindigkeit von 1 bis 5 m/s, bevorzugt zwischen 1 bis 2 m/s, aufweisen kann. Die Durchströmungsgeschwindigkeit kann insbesondere auch von dem Innendurchmesser des Gehäuses und/oder von der erreichten konstruktiven Interferenz in der UV-Behandlungskammer (das heißt im Innenraum des Gehäuses) abhängen.

Bevorzugt ist der Ventilator stromaufwärts zu der UV-Behandlungskammer bzw. zu der Strahlungsquelle und/oder dem Innenraum des Gehäuses angeordnet, um den Mediumstrom vom Einlass zu dem Auslass zu erzeugen.

Der Mediumstrom kann zumindest im Wesentlichen laminar durch den Innenraum des Gehäuses geführt werden. Alternativ oder zusätzlich kann aber auch vorgesehen sein, dass der in dem Gehäuse befindliche bzw. das Gehäuse durchströmende Mediumstrom eine turbulente Strömung ausbildet.

Des Weiteren kann mindestens eine Strahlungsquelle UV-Strahlung in einem Wellenlängenbereich von wenigstens 240 bis 300 nm, bevorzugt in einem Wellenlängenbereich von 250 bis 285 nm, weiter bevorzugt von 270 bis 280 nm und insbesondere von 254 nm +/- 10 % und/oder von 278 nm +/- 10 %, emittieren. Bei einer UV-C-Strahlung mit einer Wellenlänge von 254 nm +/- 10 % kann insbesondere eine hohe Vireninaktivierung erreicht werden. UV-Strahlung mit einer Wellenlänge in der vorgenannten Größenordnung ermöglicht erfindungsgemäß die Abtötung der Mikroorganismen in dem Mediumstrom.

Vorzugsweise ist vor dem Einlass ein Vorfilter angeordnet. Der Vorfilter kann bevorzugt derart ausgebildet sein, dass Partikel mit einem Durchmesser größer 1 μm, bevorzugt größer 0,5 μm, aus dem Mediumstrom herausgefiltert werden. Somit können insbesondere aus dem Mediumstrom solche Partikel herausgefiltert werden, die andernfalls eine sogenannte "Schattenbildung" in der UV-Behandlungskammer bei der entstehenden Wechselwirkung mit der UV-Strahlung erzeugen würden. Letztlich ist in diesem Zusammenhang relevant, dass die UV-Strahlung in einer Größenordnung zwischen 0,2 bis 0,3 μm liegt. Da die vorgenannten Partikel, beispielsweise Staubpartikel oder Pollen oder dergleichen, jedoch einen größeren Durchmesser als die Wellenlänge aufweisen, kann die Wellenlänge insbesondere nicht die Partikel mit einem Durchmesser von größer als 1 um passieren. Ein Bakterium kann beispielsweise einen Durchmesser von circa 0,3 μm aufweisen. Erfindungsgemäß ist festgestellt worden, dass der Schatteneffekt vor Partikeln mit einem Durchmesser unter 0,5 μm, insbesondere zwischen 0,3 μm bis 0,5 μm, zwar vorhanden ist, jedoch noch die erreichte Abtötung der Mikroorganismen toleriert werden kann. So können auch diejenigen Mikroorganismen mit einem Durchmesser größer als die Wellenlänge der UV-Strahlung ebenfalls unschädlich gemacht werden, da auch auf sie die UV-Strahlung trifft.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, auch wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt:

Fig. 1     eine schematische perspektivische Darstellung einer nicht-erfindungsgemäßen Bestrahlungsvorrichtung,

Fig.2     eine schematische Vorderansicht der in Fig. 1 dargestellten Bestrahlungsvorrichtung,

Fig. 3     eine schematische Draufsicht auf die in Fig. 1 dargestellte Bestrahlungsvorrichtung,

Fig. 4     eine schematische Seitenansicht der in Fig. 1 dargestellten Bestrahlungsvorrichtung,

Fig. 5     eine schematische perspektivische Darstellung einer weiteren Ausführungsform der nicht-erfindungsgemäßen Bestrahlungsvorrichtung,

Fig. 6     eine schematische perspektivische Darstellung einer weiteren Ausführungsform der nicht-erfindungsgemäßen Bestrahlungsvorrichtung,

Fig. 7     eine schematische perspektivische Darstellung einer weiteren Ausführungsform der nicht-erfindungsgemäßen Bestrahlungsvorrichtung,

Fig. 8      eine schematische perspektivische Darstellung eines erfindungsgemäßen Gehäuses,

Fig. 9      eine schematische perspektivische Darstellung einer Ausführungsform einer erfindungsgemäßen Bestrahlungsvorrichtung,

Fig. 10      eine schematische Seitenansicht auf die in Fig. 9 dargestellte Bestrahlungsvorrichtung,

Fig. 11      eine weitere schematische Seitenansicht auf die in Fig. 9 dargestellte Bestrahlungsvorrichtung,

Fig. 12      eine schematische Draufsicht auf eine erfindungsgemäße erste Halteeinheit,

Fig. 13      eine schematische Darstellung einer erfindungsgemäßen Halteeinrichtung,

Fig. 14      eine schematische Darstellung der Anordnung wenigstens einer Strahlungsquelle im Reflektor,

Fig. 15      eine schematische Darstellung der Anordnung von zwei Strahlungsquellen im Reflektor,

Fig. 16      eine schematische Darstellung einer weiteren Ausführungsform eines ersten Haltemittels und

Fig. 17      eine schematische Darstellung der erfindungsgemäßen Ausrichtung einer Strahlungsquelle.

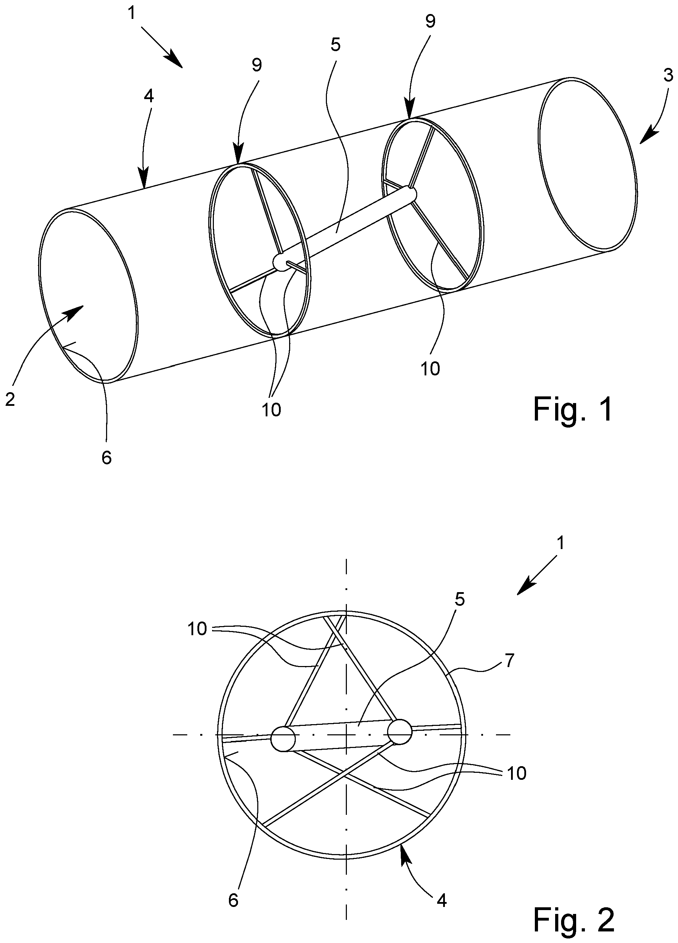

Fig. 1 zeigt eine nicht erfindungsgemäße Bestrahlungsvorrichtung 1 zur UV-Strahlung, insbesondere zur

UV-C-Bestrahlung, eines die Bestrahlungsvorrichtung 1 durchströmenden Mediums. Als Medium kann Wasser oder Luft vorgesehen sein. In weiteren Ausführungsformen kann als Medium auch ein Gasgemisch und/oder ein Dampfgemisch verwendet werden.

Die Bestrahlungsvorrichtung 1 wird insbesondere zur Inaktivierung bzw. Abtötung von in dem Medium befindlichen Mikroorganismen, wie Bakterien, Keime, Schimmel und/oder Viren, eingesetzt.

Die Bestrahlungsvorrichtung 1 weist einen Einlass 2 und einen Auslass 3 auf. Der Einlass 2 dient zum Einlass des Mediums und der Auslass 3 zum Auslass des Mediums aus der Bestrahlungsvorrichtung 1. Die Bestrahlungsvorrichtung 1 umfasst ferner ein Gehäuse 4, das den Einlass 2 und den Auslass 3 aufweist und das von dem Medium durchströmt wird. In dem Gehäuse 4 ist wenigstens eine Strahlungsquelle 5 angeordnet. Die Strahlungsquelle 5 kann mit dem Gehäuse 4 verbunden und/oder befestigt und/oder an dem Gehäuse 4 gelagert sein. Fig. 1 zeigt, dass die zwei Stirnseiten der Strahlungsquelle 5 an dem Gehäuse 4 befestigt sind.

Das Gehäuse 4 ist an der der Strahlungsquelle 5 zugewandten Innenseite 6 zumindest bereichsweise, vorzugsweise vollflächig, reflektierend mit einem Reflexionsgrad für die von der Strahlungsquelle 5 emittierte UV-Strahlung, insbesondere der UV-C-Strahlung, von wenigstens 0,6 ausgebildet. In dem in Fig. 1 dargestellten Ausführungsbeispiel ist vorgesehen, dass der Reflexionsgrad wenigstens 0,7, bevorzugt wenigstens 0,8, beträgt.

Die Innenseite 6 des Gehäuses 4 kann auch Bestandteil einer Innenwandung 7 sein. Die Innenwandung 7 kann in weiteren, nicht dargestellten Ausführungsbeispielen als austauschbare Komponente bzw. als aus dem Gehäuse 4 entnehmbare Komponente ausgebildet sein. Die Innenwandung 7 und/oder die Innenseite 6 ist im dargestellten Ausführungsbeispiel ein Reflektor, der zur Reflektion der von der Strahlungsquelle 5 ausgesendeten Strahlung ausgebildet ist.

Ferner ist in Fig. 1 dargestellt, dass die Strahlungsquelle 5 mit einer Grund-Halteeinrichtung 9 an der Innenseite 6 des Gehäuses 4 befestigt ist. Die Grund-Halteeinrichtung 9 kann in diesem Zusammenhang eine Mehrzahl von Grund-Haltemitteln 10 aufweisen. Die Grund-Haltemittel 10 sind im dargestellten Ausführungsbeispiel als strebenförmige Halterungen ausgebildet. In dem in Fig. 1 dargestellten Ausführungsbeispiel ist gezeigt, dass die Stirnseite bzw. der stirnseitige Bereich der Strahlungsquelle 5 mit wenigstens zwei, insbesondere drei, Grund-Haltemitteln 10 der Grund-Halteeinrichtung 9 an der Innenseite 6 befestigt werden kann.

Insbesondere kann die Strahlungsquelle 5 drehfest über die Grund-Halteeinrichtung 9 an dem Gehäuse 4 fixiert sein.

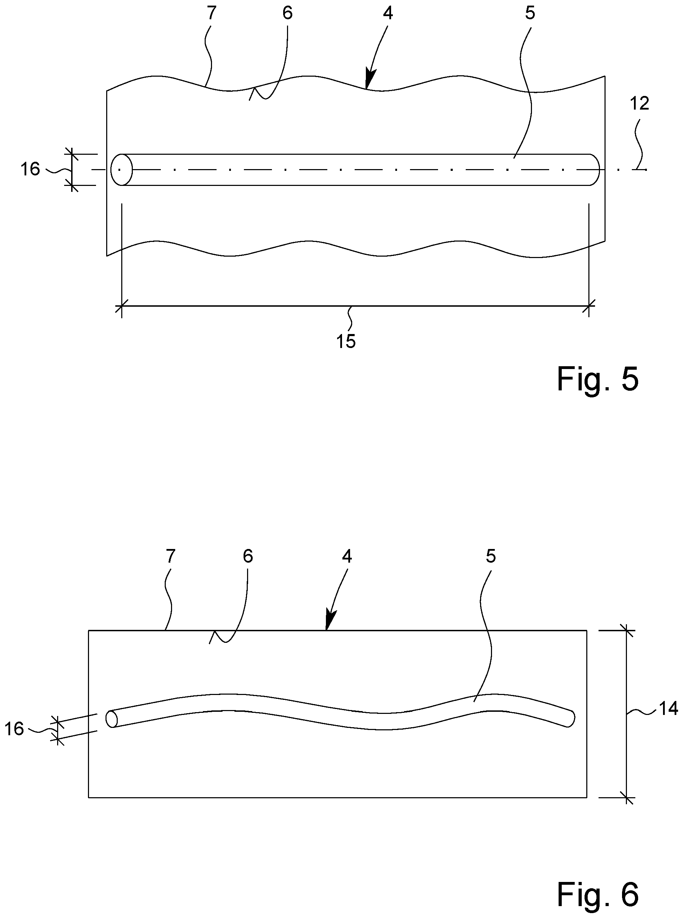

Die Innenseite 6 weist bei dem in Fig. 5 dargestellten Ausführungsbeispiel einen Reflexionsgrad von wenigstens 0,7 auf. In weiteren Ausführungsformen kann der Reflexionsgrad wenigstens 0,8, insbesondere wenigstens 0,9, betragen. Der Reflexionsgrad gibt letztlich denjenigen Anteil der von der Strahlungsquelle 5 emittierten Strahlung an, der an der Innenseite 6 reflektiert wird.

Wenngleich es nicht dargestellt ist, kann bei weiteren Ausführungsformen vorgesehen sein, dass die Innenseite 6 des Gehäuses 4 und/oder die Innenwandung 7 des Gehäuses 4 mit einer UV-Strahlung reflektierenden Beschichtung zumindest bereichsweise, insbesondere vollständig, beschichtet ist. Die Innenwandung 7 kann dabei als entnehmbare Komponente oder als Reflektor ausgebildet sein.

Des Weiteren ist nicht dargestellt, dass die Innenwandung 7 und/oder der Innenseite 6 als Material Metall aufweisen kann und/oder daraus bestehen kann. In weiteren Ausführungsformen kann die Innenwandung 7 ein Blech, insbesondere ein Aluminiumblech und/oder ein verzinktes Blech und/oder ein Edelstahlblech, sein.

Fig. 5 zeigt, dass die Innenwandung 7 des Gehäuses 4 gewellt ausgebildet ist.

Bei dem in Fig. 6 dargestellten Ausführungsbeispiel ist die Innenwandung 7 des Gehäuses 4 bzw. auch die Innenseite 6 des Gehäuses 4 eben - also flach und ohne Erhebungen - ausgebildet.

Fig. 1 zeigt, dass die Innenwandung 7 des Gehäuses 4 zylinderförmig ausgebildet ist.

Letztlich kann die Innenwandung 7 und/oder der Innenseite 6 in weiteren Ausführungsformen auch eine Mehrzahl von Erhebungen und/oder Vertiefungen aufweisen.

Die in Fig. 1 dargestellte Innenwandung 7 ist ferner rotationssymmetrisch ausgebildet. Es versteht sich jedoch, dass die Innenwandung 7 nicht zwingend symmetrisch ausgebildet sein muss.

Aus den Fig. 2 bis 4 geht anschaulich hervor, dass die Strahlungsquelle 5 derart in dem Gehäuse 4 bzw. in der Behandlungskammer 8 positioniert ist, dass sich keine symmetrische Anordnung der Strahlungsquelle 5 in dem Gehäuse 4 ergibt.

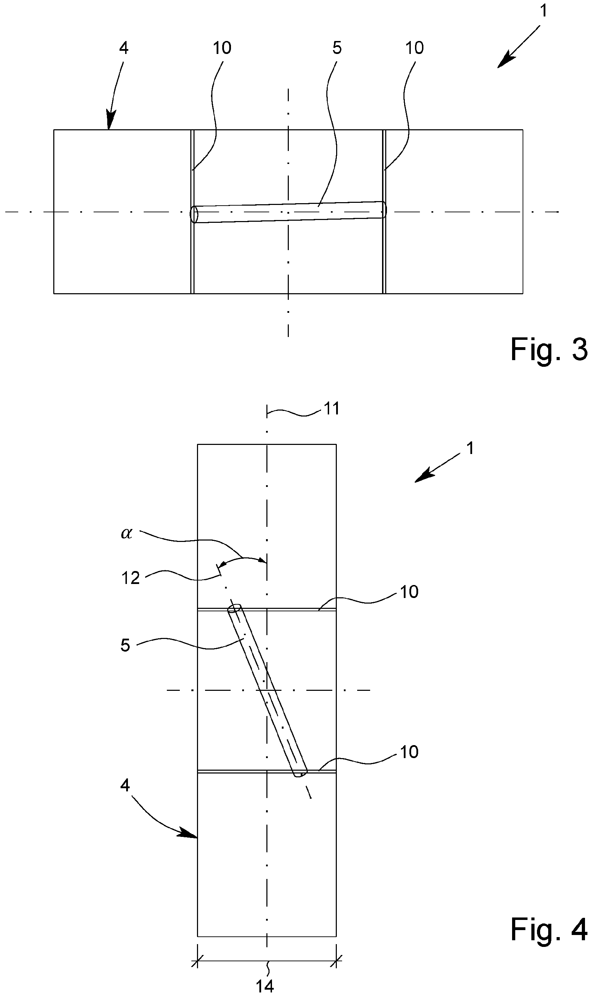

In Fig. 4 ist dargestellt, dass die Längsachse 11 des Gehäuses 4 versetzt zu der Längsachse 12 der Strahlungsquelle 5 ausgerichtet ist. Die Längsachse 11 des Gehäuses 4 ist in Richtung der größten Erstreckung angeordnet. Im dargestellten Ausführungsbeispiel entspricht die Längsachse 11 auch der Symmetrieachse des Gehäuses 4. Die Längsachse 12 der Strahlungsquelle 5 ist ebenfalls in Längserstreckung der Strahlungsquelle 5 angeordnet. Im dargestellten Ausführungsbeispiel sind die Längsachsen 11 und 12 zueinander schräg ausgerichtet. Sie können einen Winkel α zwischen 3° bis 30° einschließen, wie ebenfalls die Fig. 4 verdeutlicht.

Fig. 3 zeigt, dass sich in der Draufsicht keine Verschiebung der Achsen der Strahlungsquelle 5 und des Gehäuses 4 ergibt. Bei dem in den Fig. 1 bis 4 dargestellten Ausführungsbeispiel ist die Strahlungsquelle 5 letztlich relativ zum Gehäuse 4 in einer Ebene verschoben.

Fig. 1 zeigt, dass die Strahlungsquelle 5 dezentral in Bezug zum Gehäuse 4 angeordnet ist.

Die Grund-Halteeinrichtung 9 ist derart ausgebildet, dass die schräge Anordnung der Längsachsen 11 und 12 zueinander sichergestellt werden kann. In weiteren Ausführungsformen kann vorgesehen sein, dass eine Einstellung bzw. Veränderung der Schrägstellung der Strahlungsquelle 5 über eine Verstellung der Grund-Halteeinrichtung 9 erfolgen kann. Die Grund-Halteeinrichtung 9 kann demzufolge in weiteren Ausführungsformen einstellbar bzw. zur Verstellung der Strahlungsquelle 5 ausgebildet sein.

Die Schrägstellung der Strahlungsquelle 5 kann so ausgewählt sein, dass die gewünschten Interferenzeffekte durch konstruktive Interferenz erreicht werden können.

Des Weiteren zeigt die Fig. 4, dass die Längsachse 11 des Gehäuses 4 nicht koaxial zur Längsachse 12 der Strahlungsquelle 5 verläuft. Des Weiteren sind die Längsachsen 11 und 12 auch nicht parallel zueinander.

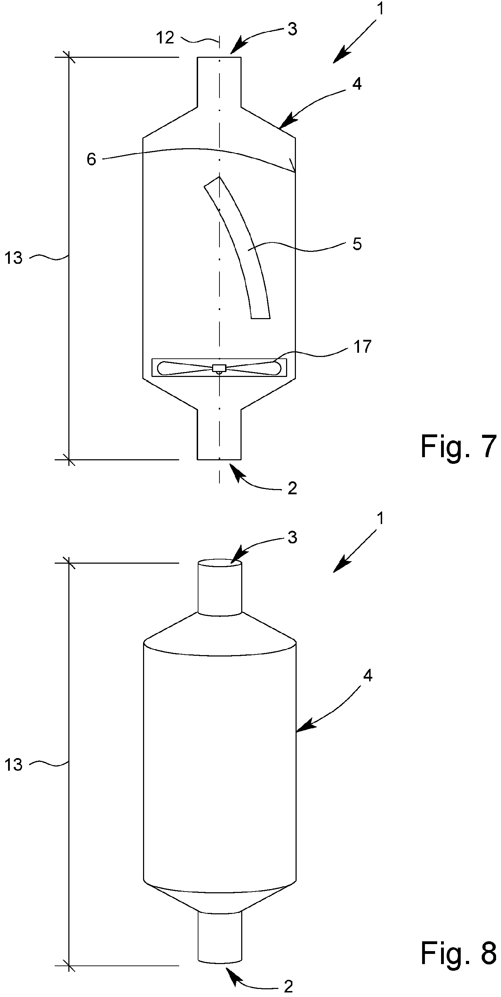

Das Gehäuse 4 kann eine Länge 13 zwischen 30 bis 200 cm aufweisen. Die Länge 13 ist schematisch in den Fig. 7 und 8 dargestellt. Das Gehäuse 4 kann einen Durchmesser 14, insbesondere einen Innendurchmesser, zwischen 8 bis 30 cm aufweisen.

Nicht dargestellt ist, dass die Strahlungsquelle 5 eine Mehrzahl von Leuchtmitteln, vorzugsweise LEDs, aufweisen kann. Dabei können die mehreren Leuchtmittel der Strahlungsquelle 5 in einem "array", insbesondere unmittelbar nebeneinander, angeordnet sein, so dass sich insbesondere eine langgestreckte Form der Strahlungsquelle 5 ergibt.

In dem dargestellten, nicht erfindungsgemäßen Ausführungsbeispiel ist vorgesehen, dass die Strahlungsquelle 5 eine langgestreckte und stabförmige Form aufweist.

Die Länge 15 der Strahlungsquelle 5 kann zwischen 5 cm bis 30 cm liegen.

Der Durchmesser 16 der Strahlungsquelle 5 kann in weiteren Ausführungsformen zwischen 1 bis 20 cm liegen, insbesondere 5 cm +/- 1 cm betragen.

Nicht dargestellt ist ferner, dass eine Mehrzahl von Strahlungsquellen 5 in dem Gehäuse 4 angeordnet ist. Die von den Strahlungsquellen 5 emittierten Strahlungen, insbesondere die UV-C-Strahlung, können sich demzufolge gegenseitig beeinflussen bzw. miteinander wechselwirken. Durch die mehreren Strahlungsquellen 5 kann letztlich die Leistung der bereitgestellten UV-Strahlung erhöht werden. Die Strahlungsquellen 5 können derart zueinander (relativ) angeordnet werden, dass die geforderten Interferenzeigenschaften erreicht werden können. So können in der Behandlungskammer 8 zwischen 2 bis 10 Strahlungsquellen 5 angeordnet sein.

Zudem ist nicht dargestellt, dass bei einer Mehrzahl von Strahlungsquellen 5 diese zumindest bereichsweise überlappend in dem Gehäuse 4 angeordnet sind. Die Länge des Überlappungsbereichs kann dabei zwischen 30 bis 80 % der Länge 15 wenigstens einer Strahlungsquelle 5 entsprechen. Auch können die Strahlungsquellen 5 zueinander windschief und/oder schräg und/oder versetzt angeordnet sein, insbesondere sind die Längsachsen 12 der Strahlungsquellen 5 zueinander versetzt und/oder schräg angeordnet. Die Anordnung der Strahlungsquellen 5 kann im Hinblick auf das erzeugte Interferenzbild erfolgen.

Fig. 7 zeigt, dass die Bestrahlungsvorrichtung 1 einen Ventilator 17 zum Erzeugen eines Mediumstromes vom Einlass 2 zum Auslass 3 umfasst. Im dargestellten Ausführungsbeispiel ist der Ventilator 17 hinter dem Einlass 2 angeordnet. In weiteren Ausführungsformen kann der Ventilator 17 auch vor dem Einlass 2 angeordnet sein.

Die von der Strahlungsquelle 5 bereitgestellte UV-Strahlung kann in einem Wellenlängenbereich von wenigstens 240 nm bis 300 nm, insbesondere in einem Wellenlängenbereich von 250 bis 285 nm und insbesondere bei 254 nm +/- 10 % und/oder bei 278 nm +/- 10 % liegen.

Darüber hinaus ist nicht dargestellt, dass vor dem Einlass 2 ein Vorfilter angeordnet sein kann. Der Vorfilter kann derart ausgebildet sein, dass Partikel mit einem Durchmesser von größer 1 μm, bevorzugt größer 0,5 μm, aus dem Mediumstrom gefiltert werden.

Der Mediumstrom kann eine Strömungsgeschwindigkeit zwischen 1 bis 2 m/s, insbesondere von 1,7 m/s +/- 20 %, aufweisen.

In weiteren Ausführungsformen kann die Strahlungsquelle 5 eine Leistung zwischen 50 bis 500 W, bevorzugt zwischen 80 bis 200 W, aufweisen. Dabei können insbesondere wenigstens 10 %, bevorzugt zwischen 20 % bis 50 %, weiter bevorzugt zumindest im Wesentlichen zwischen 30 % bis 40 %, auf den Anteil der Leistung der Strahlungsquelle 5 für die bereitgestellte UV-C-Strahlung entfallen.

Die Strömung des Mediumstromes in der Behandlungskammer 8 kann laminar und/oder turbulent ausgebildet sein. Letztlich kann die Strömung auch durch den Ventilator 17 beeinflusst werden, der für die Durchströmung des Gehäuses 4 sorgt.

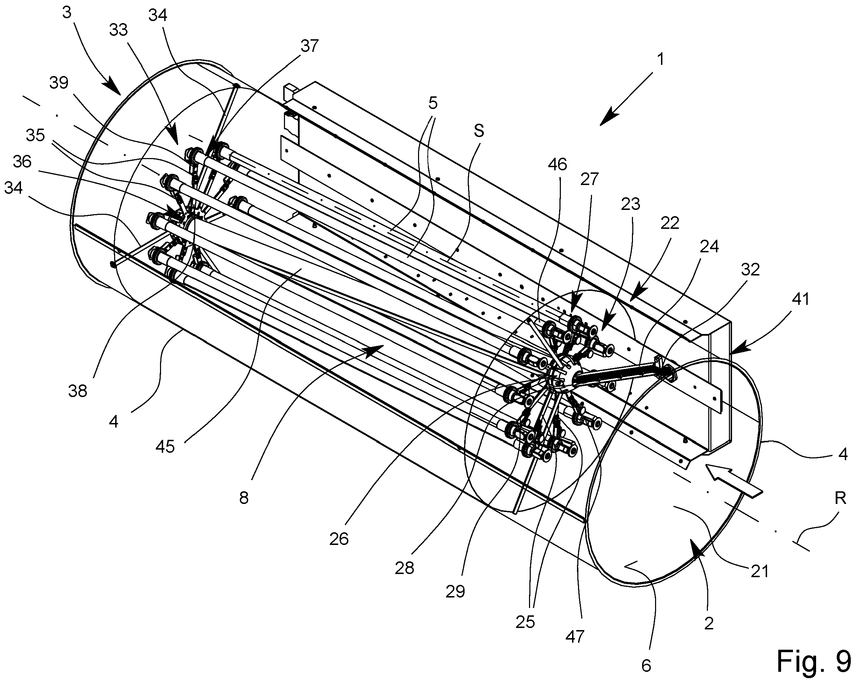

In den Fig. 9 bis 16 ist eine erfindungsgemäße Ausführungsform einer Bestrahlungsvorrichtung 1 dargestellt. In diesem Zusammenhang versteht es sich, dass Ausführungen, wie sie zur Bestrahlungsvorrichtung 1 nach den Fig. 1 bis 8 gemacht worden sind, auch für die nachfolgend beschriebene erfindungsgemäße Ausführungsform der Bestrahlungsvorrichtung 1 gelten können - jedoch nicht gelten müssen. Zur Vermeidung von unnötigen Wiederholungen wird darauf verzichtet, auf die diesbezüglichen einzelnen Merkmale erneut einzugehen.

Die Fig. 9 zeigt eine Bestrahlungsvorrichtung 1, die zur UV-Bestrahlung, insbesondere UV-C-Bestrahlung, eines die Bestrahlungsvorrichtung 1 durchströmenden Mediums ausgebildet ist. Das Medium kann ein Fluid oder ein Gas sein. Insbesondere ist als Medium Wasser oder Luft vorgesehen. Die Bestrahlungsvorrichtung 1 wird zur Inaktivierung von in dem Medium befindlichen Mikroorganismen, wie Bakterien, Keime, Schimmel und/oder Viren, verwendet. Insbesondere wird die Bestrahlungsvorrichtung 1 zur Inaktivierung von Corona-Viren verwendet. Als Corona-Viren werden insbesondere SARS-CoV-2-Viren verstanden.

Die Bestrahlungsvorrichtung 1 weist ein Gehäuse 4 auf, das für das Medium einen Einlass 2 und einen Auslass 3 aufweist. In Fig. 9 ist die Durchströmungsrichtung des Mediums schematisch über Strömungs-Pfeile dargestellt.

Mehr als eine Strahlungsquelle 5 ist in dem Gehäuse 4, nämlich im Inneren des Gehäuses 4, angeordnet. Das Innere des Gehäuses 4 umfasst die Behandlungskammer 8, in der die Strahlungsquelle(n) 5 angeordnet ist/sind.

Die Strahlungsquelle 5 dient zur Bestrahlung des das Gehäuse 4 durchströmenden Mediums.

In der Ausführungsform nach Fig. 9 ist eine Mehrzahl von Strahlungsquellen 5 vorgesehen.

Das Gehäuse 4 weist einen Reflektor 21 auf. Die Innenseite 6 des Reflektors 21 bildet auch die Innenseite 6 des Gehäuses 4. In den dargestellten Ausführungsformen gemäß den Fig. 9 bis 11 ist aus Veranschaulichungsgründen der Reflektor 21 "durchsichtig" dargestellt.

Insbesondere ist der Reflektor 21 als ein Aluminium-Blech ausgebildet, das in einem entsprechenden Profil eingefasst bzw. gehalten werden kann.

Die Innenseite 6 ist zumindest bereichsweise, vorzugsweise vollflächig, reflektierend mit einem Reflexionsgrad für die von der Strahlungsquelle 5 emittierte UV-Strahlung von größer als 0,6 ausgebildet. Insbesondere ist die Innenseite 6 so ausgebildet, dass eine gerichtete, direkte Reflektion der Strahlung erfolgen kann. Hierzu ist die Innenseite 6 insbesondere glatt und eben ausgebildet.

Die Strahlungsquelle 5 wird mit einer Halteeinrichtung 22 gehalten und/oder fixiert. Die Halteeinrichtung 22 ist, vorzugsweise lösbar, mit dem Gehäuse 4 und/oder dem Reflektor 21 verbunden.

Fig. 9 zeigt, dass die Halteeinrichtung 22 derart ausgebildet ist, dass die Mittelachse S der wenigstens einen Strahlungsquelle 5 einen Winkel $\alpha$ zur Mittelachse R des Reflektors 21 einschließt.

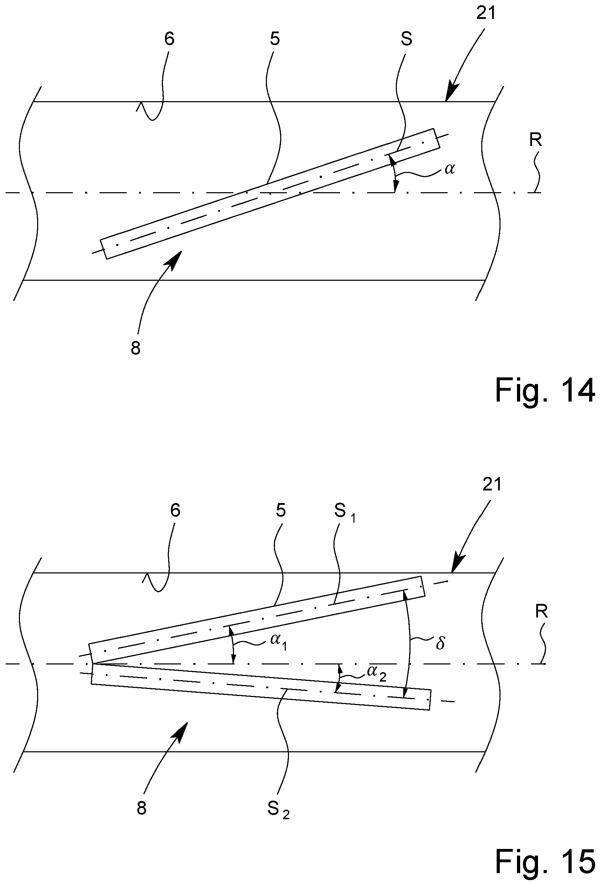

Fig. 14 zeigt schematisch, dass die Strahlungsquelle 5 so angeordnet ist, dass zwischen den Mittelachsen S und R ein Winkel $\alpha$ eingeschlossen wird. Aus Veranschaulichungsgründen ist in Fig. 14 die Halteeinrichtung 22 nicht näher dargestellt.

Bei der in Fig. 14 dargestellten Ausführungsform liegt der eingeschlossene Winkel $\alpha$ zwischen der Mittelachse S der wenigstens einen Strahlungsquelle 5 und der Mittelachse R der Reflektors 21 zwischen $\arcsin((0{,}2 \cdot D/L)$ und $\arcsin((4 \cdot D)/L)$. Insbesondere liegt der Winkel $\alpha$ zwischen $2° \pm 0{,}5°$. Um die Schrägstellung der Strahlungsquelle 5 aus schematischen Gründen besser darzustellen, ist in den dargestellten Ausführungsformen nach Fig. 14 und 15 der Winkel $\alpha$ bewusst größer gewählt worden. Es versteht sich jedoch, dass diese Figuren als schematische Darstellungen zu verstehen sind und nicht die tatsächlichen Größenverhältnisse widerspiegeln.

Ferner versteht es sich, dass der Winkel $\alpha$ insbesondere in der vorgenannten Größenordnung liegt.

Vorzugsweise liegt der Winkel $\alpha$ zwischen $\arcsin(D/L)$ und $\arcsin((2 \cdot D)/L)$. Somit beträgt der insgesamt eingenommen Schrägversatz insbesondere zwischen D und 2D.

Dabei gibt D den, insbesondere maximalen und/oder mittleren, Durchmesser der Strahlungsquelle 5 und L die Länge der Strahlungsquelle 5 an.

Die in den dargestellten Ausführungsformen gezeigten Strahlungsquellen 5 sind insbesondere als LED-Strahler ausgebildet.

Die Strahlungsquellen 5 sind ferner stabförmig bzw. zylinderförmig sowie langgestreckt ausgebildet. Die Längserstreckung der Strahlungsquelle 5 verläuft dabei zumindest im Wesentlichen in Richtung Längserstreckung des Reflektors 21 - unter Berücksichtigung der zuvor diskutierten Schrägstellung der Strahlungsquelle(n) 5. Somit wird bevorzugt keine orthogonale Anordnung der Strahlungsquelle 5 in Bezug zur Mittelachse R des Reflektors 21 vorgesehen.

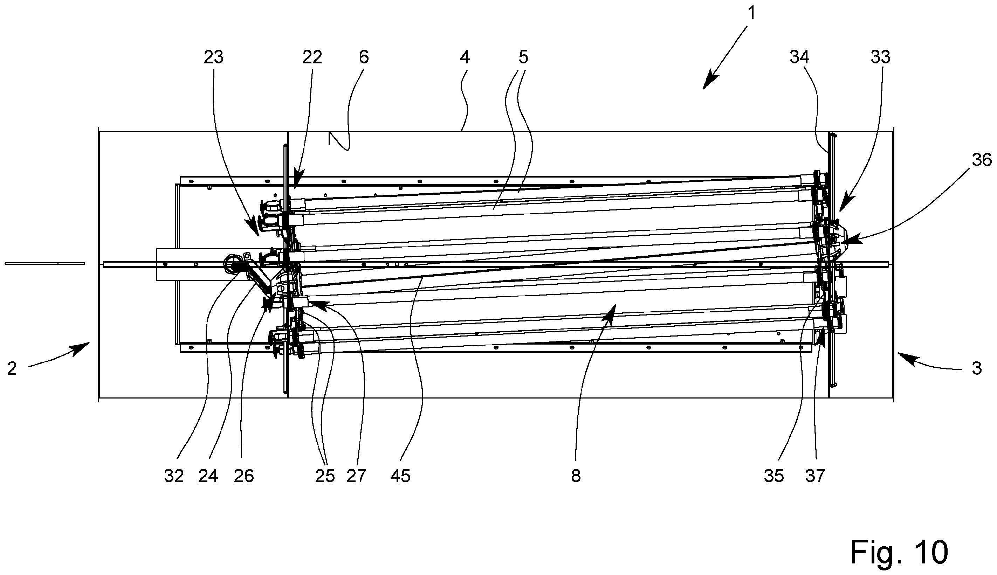

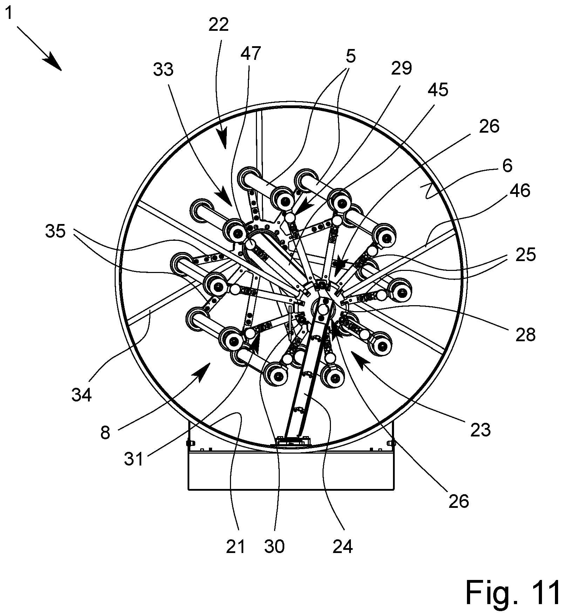

Wie zuvor erläutert, zeigen die Fig. 9 bis 11, dass eine Mehrzahl von Strahlungsquellen 5 an der Halteeinrichtung 22 gehalten und/oder fixiert sind. Die Fig. 9 und 10 zeigen entsprechende Seitenansichten auf die in Fig. 9 dargestellte Bestrahlungsvorrichtung 1. So zeigt Fig. 11 den Einlass 2, wobei Fig. 10 die schräge Anordnung der Strahlungsquellen 5 durch die entsprechende Seitenansicht der Längsseite verdeutlicht.

In diesem Zusammenhang versteht es sich, dass in weiteren Ausführungsformen auch eine Mehrzahl von Halteeinrichtungen 22 vorgesehen sein kann, wobei an den jeweiligen Halteeinrichtungen 22 jeweils wenigstens eine Strahlungsquelle 5, bevorzugt eine Mehrzahl von Strahlungsquellen 5, befestigt sein kann. Diese Halteeinrichtungen 22 können dabei untereinander und/oder nebeneinander, insbesondere beabstandet zueinander, angeordnet sein. Besonders bevorzugt ist jedoch, dass eine einzige Halteeinrichtung 22 vorgesehen ist.

Die an der Halteeinrichtung 22 befestigten Strahlungsquellen 5 können insgesamt auch als "Lampenpaket" bzw. Strahlungseinheit bezeichnet werden.

Der Einlass 2 und der Auslass 3 können auch an anderen Stellen des Gehäuses 4 angeordnet sein. Letztlich dient der Einlass 2 zur Einführung des Mediums in die Behandlungskammer 8, während der Auslass 3 den Austritt des Mediums aus der Bestrahlungsvorrichtung 1 ermöglicht. Grundsätzlich kann erfindungsgemäß auch vorgesehen sein, dass eine Mehrzahl von Einlässen 2 und/oder eine Mehrzahl von Auslässen 3 vorhanden ist.

In der dargestellten Ausführungsform ist es so, dass in Längsrichtung des Reflektors 21 an der Halteeinrichtung 22 jeweils nur eine Strahlungsquelle 5 angeordnet ist. Die weiteren Strahlungsquellen 5 sind ebenfalls zumindest im Wesentlichen in Längsrichtung ausgerichtet. Nicht dargestellt ist, dass bei einer weiteren Ausführungsform auch vorgesehen sein kann, dass wenigstens zwei Strahlungsquellen 5 in Längsrichtung des Reflektors 21 hintereinander an einer Halteeinrichtung 22 angeordnet sein können. Auch kann eine Strahlungsquelle 5 mehrteilig ausgebildet sein.

Bei der in Fig. 10 dargestellten Ausführungsform ist es so, dass jede Mittelachse S jeder Strahlungsquelle 5 einen Winkel $\alpha$ zu der Mittelachse R des Reflektors 21 einschließt. **In** Fig. 15 ist schematisch dargestellt, dass die Mittelachsen $S_1$ und $S_2$ jeweils einen Winkel $\alpha_1$ und $\alpha_2$ zur Mittelachse R des Reflektors 21 einschließen.

Unter der Mittelachse wird diejenige Achse verstanden, die eine annähernde Symmetrieachse des Körpers bildet. Allerdings werden auch nicht-symmetrische Körper berücksichtigt. **In** diesem Fall kann die Mittelachse insbesondere durch den Schwerpunkt des Körpers und in Längserstreckung des Körpers verlaufen. Auch Abweichungen zur Mittelachse von $\pm$ 10% werden erfindungsgemäß noch unter der "Mittelachse" subsummiert.

**In** Fig. 11 ist schematisch dargestellt, dass die Mittelachsen S der Strahlungsquellen 5 zueinander zumindest im Wesentlichen parallel angeordnet sind.

**In** Fig. 15 ist schematisch dargestellt, dass wenigstens zwei Mittelachsen $S_1$ und $S_2$ zueinander versetzt, insbesondere schräg, angeordnet sind. Dabei kann der einge**schlossene** Winkel $\delta$ zwischen wenigstens zwei Strahlungsquellen 5 zwischen 1° bis 50°, insbesondere zwischen 10° bis 40°, betragen.

Insbesondere können die Mittelachsen S der Strahlungsquellen 5 zueinander auch schräg und/oder windschief angeordnet sein.

Bei der in Fig. 9 dargestellten Halteeinrichtung 22 ist vorgesehen, dass diese derart ausgebildet ist, dass die Strahlungsquelle 5 bzw. die Strahlungsquellen 5 lösbar mit der Halteeinrichtung 22 verbunden sind.

Die Strahlungsquellen 5 können zueinander gleich beabstandet sein. Es kann jedoch auch vorgesehen sein, dass die Mittelachsen R einen unterschiedlichen Winkel $\alpha_1$, $\alpha_2$ zu der Mittelachse R des Reflektors einschließen, wie dies schematisch beispielsweise in Fig. 15 dargestellt ist. Auch bei der in Fig. 15 dargestellten Ausführungsform ist vorgesehen, dass die Winkel $\alpha_1$ und $\alpha_2$ - aus schematischen Darstellungszwecken - bewusst "größer" dargestellt sind, um letztlich das Prinzip zu verdeutlichen.

Fig. 9 zeigt, dass die Halteeinrichtung 22 eine erste Halteeinheit 23 aufweist. Die erste Halteeinheit 23 ist über ein erstes Verbindungsmittel 24 der Halteeinrichtung 22 lösbar mit dem Gehäuse 4 und dem Reflektor 21 verbunden. Zur weiteren Stabilität der ersten Halteeinheit 23 sind darüber hinaus noch Haltestreben 46 vorgesehen, die jeweils mit dem Gehäuse 4 und/oder dem Reflektor 21 verbunden sind. Die Haltestreben 46 können als Bestandteil des ersten Verbindungsmittels 24 angesehen werden.

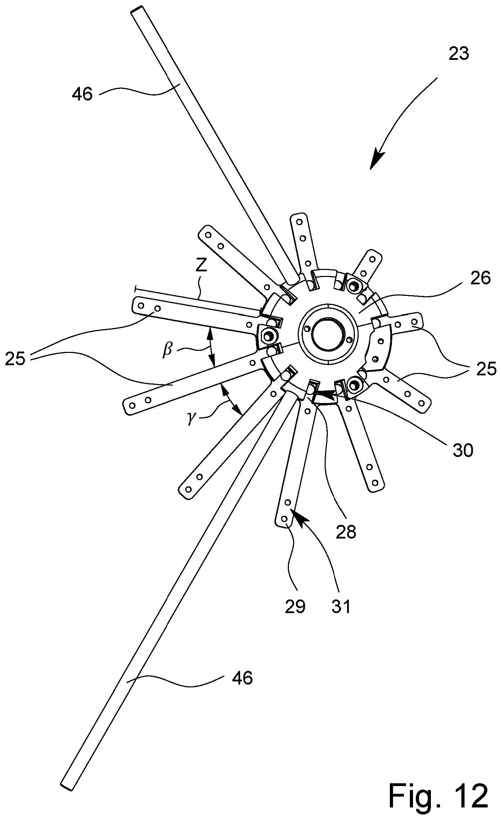

Schematisch sind die Haltestreben 46 darüber hinaus auch in Fig. 12 dargestellt. Fig. 12 zeigt die erste Halteeinheit 23 ohne entsprechende Befestigungsmittel 47 für die Strahlungsquellen 5.

Fig. 12 zeigt, dass die erste Halteeinheit 23 erste Haltemittel 25 aufweist, wobei die ersten Haltemittel 25 insbesondere als stegförmige Haltearme ausgebildet sind. Die ersten Haltemittel 25 können zumindest bereichsweise zueinander beabstandet sein, wie dies aus Fig. 12 ersichtlich ist. Die Beabstandung zwischen den ersten Haltemitteln 25 kann ferner variieren. Ebenfalls kann der eingeschlossene Winkel $\beta$, $\gamma$ zwischen zwei unmittelbar benachbarten ersten Haltemitteln 25 variieren. Die **Winkel** $\beta$, $\gamma$ beziehen sich insbesondere auf die Mittelachse der ersten Haltemittel 25.

Zur Befestigung der Strahlungsquellen 5 kann das erste Haltemittel 25 Befestigungsmittel 47 aufweisen. Die Befestigungsmittel 47 sind schematisch in Fig. 9 dargestellt.

Als Befestigungsmittel 47 kann beispielsweise ein Clip, ein Federschenkel und/oder eine Spannklemme vorgesehen sein. Letztlich sind unterschiedliche Befestigungsmittel 47 möglich. Das Befestigungsmittel 47 ist insbesondere ein Bestandteil des ersten Haltemittels 25.

**In** Fig. 11 ist dargestellt, dass die ersten Haltemittel 25 mit einem ersten Verbindungsbereich 26 der ersten Halteeinheit 23 verbunden sind. Ausgehend von diesem Verbindungsbereich 26 stehen die ersten Haltemittel 25 ab. Die ersten Haltemittel 25 sind mit dem einen Endbereich 28 mit dem Verbindungsbereich 26 verbunden. Die ersten Haltemittel 25 umfassen ferner einen weiteren Endbereich 29, der wiederum zur Anordnung der Strahlungsquellen 5, insbesondere der stirnseitigen Endbereiche 27 der Strahlungsquelle 5, vorgesehen ist. Somit können die ersten Haltemittel 25 insbesondere als Tragarm bzw. Kragarm ausgebildet sein. Der freie Endbereich 29 kann insbesondere nicht gelagert bzw. frei angeordnet sein. Der Endbereich 28 der ersten Haltemittel 25 kann dabei an dem ersten Verbindungbereich 26 unmittelbar angeordnet sein.

Insbesondere ergibt sich eine zumindest im Wesentlichen sternförmige bzw. sonnenförmige Ausbildung der ersten Halteeinheit 23, wie dies in Fig. 12 schematisch dargestellt ist.

Der Endbereich 28 kann an dem Verbindungsbereich 26 gelagert oder mit diesem fest verbunden sein. Auch kann vorgesehen sein, dass der Verbindungsbereich 26 und der Endbereich 28 einteilig miteinander ausgebildet sind.

In der dargestellten Ausführungsform ist ferner vorgesehen, dass ein erstes Verstellmittel 30 an dem Endbereich 28 angeordnet ist. Dieses erste Verstellmittel 30 ermöglicht eine relative Verstellung zum Verbindungsbereich 26 und insbesondere eine Verstellung der an dem jeweiligen ersten Haltemittel 25 befestigten Strah**lungsquelle** 5 - und zwar eine Verstellung der Mittelachse S der Strahlungsquelle 5 in Bezug zur Mittelachse R des Reflektors 21.

Nicht näher dargestellt ist, dass die ersten Haltemittel 25 zumindest bereichsweise auch teleskopierbar ausgebildet sind.

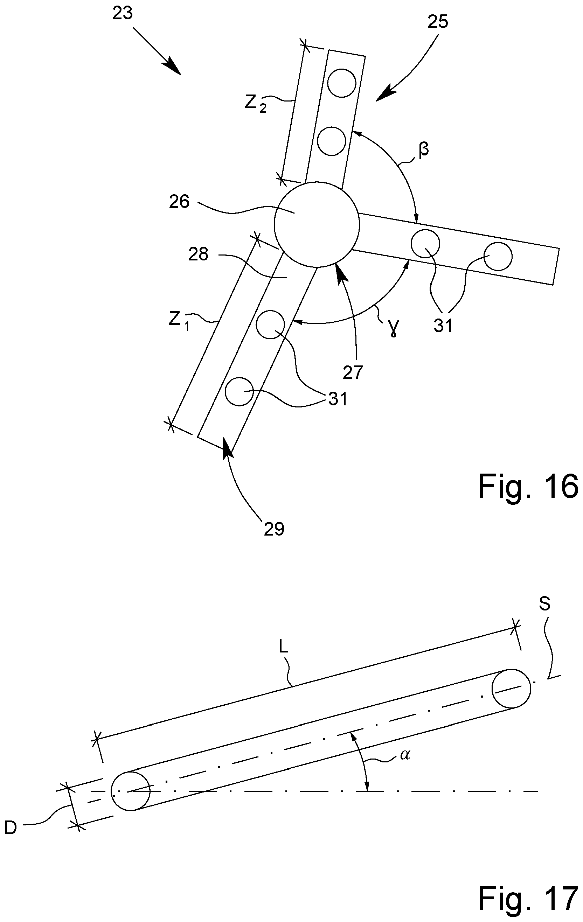

In Fig. 12 ist dargestellt, dass die ersten Haltemittel 25 eine unterschiedliche Länge Z aufweisen. Dies ist schematisch auch in Fig. 16 dargestellt.

In Fig. 11 ist schematisch dargestellt, dass die Strahlungsquelle 5 an dem einen stirnseitigen Endbereich 27 lösbar und reibschlüssig mit dem ersten Haltemittel 25, insbesondere mit dem Befestigungsmittel 47, verbunden ist.

Fig. 16 zeigt, dass die ersten Haltemittel 25 langgestreckt ausgebildet sind und dass wenigstens zwei erste Haltemittel 25 eine sich voneinander unterscheidende Länge $Z_1$, $Z_2$ aufweisen. Schematisch ist in Fig. 16 weiter dargestellt, dass je Haltemittel 25 eine Mehrzahl von Anordnungsbereichen 31 vorgesehen ist. Die Anordnungsbereiche 31 können zur Anordnung von Befestigungsmitteln 47 oder zur (unmittelbaren) Anordnung des stirnseitigen Endbereichs 27 der Strahlungsquelle 5 ausgebildet sein. So kann beispielsweise die Stirnseite der Strahlungsquelle 5 über den Anordnungsbereich 31 und somit auch über das Haltemittel 25 überstehen, insbesondere wenn der stirnseitige Endbereich 27 zumindest bereichsweise in dem Anordnungsbereich 31 aufgenommen und in diesem, vorzugsweise reibschlüssig, gehalten ist. Letztlich sind unterschiedliche Befestigungsmöglichkeiten zwischen der Strahlungsquelle 5 und dem ersten Haltemittel 25 möglich.

Die zwischen zwei unmittelbar benachbarten ersten Haltemittel 25 eingeschlossenen Winkel β, γ können insbesondere zueinander um wenigstens 5% abweichen, wie dies schematisch in Fig. 16 dargestellt ist.

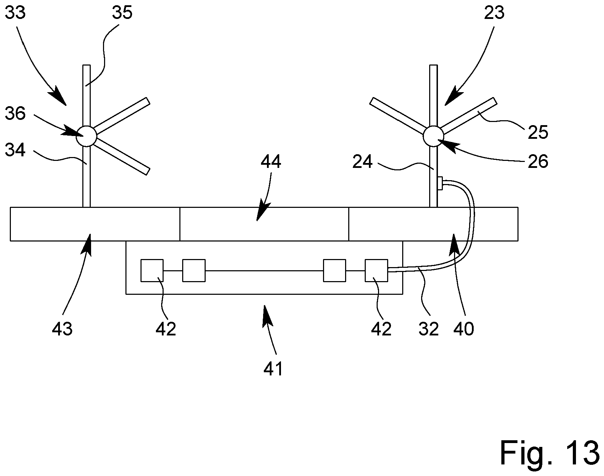

In Fig. 9 ist schematisch dargestellt, dass Energieversorgungsleitungen 32 zur Energieversorgung der Strahlungsquellen 5 vorgesehen sind. Diese Energieversorgungsleitungen 32 werden insbesondere entlang des ersten Verbindungsmittels 24 und insbesondere entlang der ersten Haltemittel 25 geführt. Die Energieversorgungsleitungen 32 können mit entsprechenden Netzteilen und/oder Vorschaltgeräten 42 verbunden sein, wie dies schematisch aus Fig. 13 ersichtlich wird. Insbesondere ist eine erste Versorgungseinrichtung 41 außerhalb des Gehäuses 4 an der Außenseite des Gehäuses 4, die der Innenseite 6 abgewandt ist, angeordnet.

Letztlich kann die erste Halteeinheit 23 zur Energiezuführung zu den Strahlungsquellen 5 ausgebildet sein.

In Fig. 9 ist dargestellt, dass eine zweite Halteeinheit 33 vorgesehen ist. Die zweite Halteeinheit 33 ist über ein zweites Verbindungsmittel 24 der Halteeinrichtung 22 lösbar mit dem Gehäuse 4 und dem Reflektor 21 verbunden.

Das zweite Verbindungsmittel 24 umfasst nach der in Fig. 9 dargestellten Ausführungsform wenigstens zwei Haltestreben, die die zweite Halteeinheit 33 mit dem Gehäuse 4 und/oder dem Reflektor 21 verbinden.

Fig. 9 zeigt, dass die zweite Halteeinheit 33 zweite Haltemittel 35 aufweist, wobei die zweiten Haltemittel 35 insbesondere als stegförmige Haltearme ausgebildet sind. Die zweiten Haltemittel 35 können zumindest bereichsweise zueinander beabstandet sein. Die Beabstandung zwischen den zweiten Haltemitteln 35 kann ferner variieren. Ebenfalls kann der eingeschlossene Winkel zwischen zwei unmittelbar benachbarten zweiten Haltemitteln 35 variieren.

Zur Befestigung der Strahlungsquellen 5 kann das zweite Haltemittel 35 Befestigungsmittel 47 aufweisen, die Befestigungsmittel 47 des zweiten Haltemittels 35 können insbesondere korrespondierend zu den Befestigungsmitteln 47 der ersten Haltemittel 25 ausgebildet sein, so dass auf die vorangegangenen Ausführungen verwiesen werden darf.

In Fig. 9 ist dargestellt, dass die zweiten Haltemittel 35 mit einem zweiten Verbindungsbereich 36 der zweiten Halteeinheit 33 verbunden sind. Ausgehend von diesem Verbindungsbereich 36 stehen die zweiten Haltemittel 35 ab. Die zweiten Haltemittel 35 sind mit dem einen Endbereich 38 mit dem Verbindungsbereich 36 verbunden. Die zweiten Haltemittel 35 umfassen ferner einen weiteren freien bzw. nicht-gelagerten Endbereich 39, der wiederum zur Anordnung der Strahlungsquellen 5, insbesondere der weiteren stirnseitigen Endbereiche 37 der Strahlungsquelle 5, vorgesehen ist.

Der Endbereich 38 kann an dem Verbindungsbereich 36 gelagert oder mit diesem fest verbunden sein. Auch kann vorgesehen sein, dass der Verbindungs-reich 36 und der Endbereich 38 einteilig miteinander ausgebildet sind.

Nicht näher dargestellt ist, dass ein zweites Verstellmittel an dem Endbereich 38 angeordnet ist. Dieses zweite Verstellmittel kann insbesondere entsprechend des ersten Verstellmittels 30 ausgebildet sein, so dass auf die Ausführungen zum ersten Verstellmittel 30 verwiesen werden darf

Nicht dargestellt ist, dass die zweiten Haltemittel 35 zumindest bereichsweise auch teleskopierbar ausgebildet sind.

Auch die zweiten Haltemittel 35 können eine unterschiedliche Länge Z aufweisen.

Nicht näher dargestellt ist, dass auch die zweiten Haltemittel 35 Anordnungsbereiche für die Strahlungsquelle(n) 5 aufweisen können. Diese Anordnungsbereiche können wie die Anordnungsbereiche 31 der ersten Halteeinheit 23 ausgebildet sein.

Das zweite Verbindungsmittel 34 ist in dem in Fig. 9 dargestellten Ausführungsbeispiel mehrteilig ausgebildet und weist eine Mehrzahl von entsprechenden Haltestreben auf. Die Haltestreben des zweiten Verbindungsmittels 34 können die zweite Halteeinheit 33 lösbar mit dem Gehäuse 4 und/oder dem Reflektor 21 verbinden.

Fig. 9 zeigt weiter, dass die erste Halteeinheit 23 mit der zweiten Halteeinheit 33 über ein Verbindungsteil 45 verbunden ist. Das Verbindungsteil 45 kann insbesondere langgestreckt ausgebildet sein und verbindet in dem dargestellten Ausführungsbeispiel den ersten Verbindungsbereich 26 mit dem zweiten Verbindungsbereich 36. Das Verbindungsteil 45 ist insbesondere steif und stabil ausgebildet. Die Außenseite des Verbindungsteils 45 kann reflektierend ausgebildet sein.

In Fig. 9 ist dargestellt, dass das Verbindungsteil 45 im Zentrum des Lampenpaketes angeordnet ist und daher von den Strahlungsquellen 5 umschlossen bzw. umgeben wird. Insbesondere steht das Verbindungsteil 45 (in Bezug zur Innenseite 6) nicht über die Strahlungsquellen 5 über.

Besonders bevorzugt ist vorgesehen, dass die zweite Halteeinheit 33 komplementär zur ersten Halteeinheit 23 ausgebildet ist, insbesondere so dass die gewünschte Schrägstellung der Strahlungsquellen 5 erreicht werden kann.

Nicht näher dargestellt ist, dass das erste Verbindungsmittel 24, das zweite Verbindungsmittel 34 und/oder die Haltestreben 46 teleskopierbar und/oder verstellbar ausgebildet sind. Eine solche Verstellung oder Teleskopierung erhöht insbesondere die Flexibilität bzw. die Anpassbarkeit der gesamten Halteeinrichtung 22.

In Fig. 13 ist eine schematische Ansicht der noch nicht ausgerichteten Halteeinrichtung 22 dargestellt. Letztlich sind die jeweiligen Strahlungsquellen 5 noch nicht an die entsprechenden Haltemittel 25, 35 angeordnet.

Fig. 13 zeigt einen dem Anschluss der Strahlungsquellen 5 über Energieversorgungsleitungen 32, die mit einer ersten Energie-Versorgungseinrichtung 41 verbunden sind, in der mehrere Vorschaltgeräte 42 angeordnet sind. Demnach kann ein modularer Aufbau der Halteeinrichtung 22 sichergestellt werden. Der modulare Aufbau kann derart angepasst werden, dass insbesondere unterschiedliche Längen für die Strahlungsquellen 5 ermöglicht werden können.

Zudem zeigt Fig. 13, dass die erste Halteeinheit 23 sowie das erste Verbindungsmittel 24 mit einem ersten Verbindungsabschnitt 40 verbunden sind. Der erste Verbindungsabschnitt 40 ist mit dem Gehäuse 4 und/oder dem Reflektor 21 lösbar verbindbar, was nicht näher dargestellt ist. Beispielsweise kann vorgesehen sein, dass der erste Verbindungsabschnitt 40 zumindest bereichsweise ein Profil zur Anordnung des Reflektors 21 aufweist, der insbesondere als Alu-Blech ausgebildet sein kann. Grundsätzlich sind aber auch weitere Ausführungsformen denkbar.

Der erste Verbindungsabschnitt 40 steht zumindest teilweise in weiteren Ausführungsformen über das Gehäuse ab bzw. über. Dabei kann ferner der erste Verbindungsabschnitt 40 außenseitig eine erste Versorgungseinrichtung 41 aufweisen. Die erste Versorgungseinrichtung 41 umfasst mehrere Vorschaltgeräte 42, wie zuvor erläutert worden ist. Die erste Versorgungseinrichtung 41 ist elektrisch mit dem ersten Verbindungsmittel 24 über die Energieversorgungsleitungen 32 verbunden. Die Energieversorgungsleitungen 32 können durch das Gehäuse 4 geführt werden, wie dies schematisch beispielsweise auch die Fig. 9 zeigt.

Darüber hinaus zeigt Fig. 13, dass die zweite Halteeinheit 33 sowie das zweite Verbindungsmittel 34 mit einem zweiten Verbindungsabschnitt 43 verbunden sind. Der zweite Verbindungsabschnitt 32 kann in weiteren Ausführungsformen ferner auch lösbar mit dem Gehäuse 4 und/oder dem Reflektor 21 verbunden sein. Darüber hinaus kann auch der zweite Verbindungsabschnitt 43 über das Gehäuse 4 in weiteren Ausführungsformen abstehen.

Der erste und zweite Verbindungsabschnitt 40, 43 kann derart ausgebildet sein, dass diese lösbar miteinander formschlüssig und/oder reibschlüssig und/oder kraftschlüssig verbindbar sind. Dazu können die Verbin-dungsabschnitte 40, 43 entsprechende Verriegelungs-konturen oder dergleichen aufweisen. In Fig. 13 ist dargestellt, dass die Verbindungsabschnitte 40, 43 über ihre Stirnseiten verbindbar sind. Entsprechende Verriegelungskonturen sind in Fig. 13 nicht näher dargestellt.

Fig. 13 zeigt, dass zum modularen Aufbau ein weiterer Verbindungsabschnitt 44 vorgesehen ist. Der weitere Verbindungsabschnitt 44 kann lösbar mit dem ersten und/oder zweiten Verbindungsabschnitt 40, 43 formschlüssig und/oder reibschlüssig und/oder kraftschlüssig verbindbar sein. Hierzu kann der weitere Ver-bindungsabschnitt 44 entsprechende Verriegelungskon-

turen, die komplementär zu den Verriegelungskonturen der unmittelbar angrenzenden Verbindungsabschnitte ausgebildet sind, aufweisen.

Nicht näher dargestellt ist, dass der erste, zweite und/oder weitere Verbindungsabschnitt 40, 43 und 44 zumindest teilweise in das Innere des Reflektors 21 hineinragen oder an die Innenseite 6 angrenzen kann - oder gegenüber dieser zurückversetzt sein kann.

Je nach Ausführungsform kann vorgesehen sein, dass zwischen 3 bis 25 Strahlungsquellen 5, erste Haltemittel 25 und/oder zweite Haltemittel 35 vorgesehen sind. Die Anzahl der Strahlungsquellen 5 kann insbesondere von der Länge des Reflektors 21, dem behandelten Volumenstrom des Mediums und dergleichen abhängen. **In** Fig. 9 ist dargestellt, dass zehn Strahlungsquellen 5 vorgesehen sind.

Nicht dargestellt ist, dass die Anzahl der ersten Haltemittel 25 und/oder der zweiten Haltemittel 35 die Anzahl der Strahlungsquellen 5 übersteigt. Es ist daher nicht zwingend erforderlich, dass an jedes Haltemittel 25 eine Strahlungsquelle 5 angeordnet werden muss. Somit kann ein "Überhang" an Haltemitteln 25, 35 vorgesehen sein.

Bei der in Fig. 9 dargestellten Ausführungsform ist vorgesehen, dass die Strahlungsquellen 5 zueinander baugleich ausgebildet sind. Grundsätzlich können auch unterschiedliche Strahlungsquellen 5 gewählt werden, sofern dies vom Nutzer gewünscht wird.

Nicht näher dargestellt ist, dass wenigstens eine, bevorzugt alle, Strahlungsquellen 5 einen Durchmesser D, insbesondere der maximale und/oder der mittlere Durchmesser D, zwischen 1 cm bis 20 cm, insbesondere zwischen 4 cm und 6 cm, aufweisen. Ferner können die Strahlungsquellen 5 eine Länge L zwischen 0,2 bis 10 m, bevorzugt zwischen 1 bis 2 m, aufweisen.

Auch der innere Durchmesser des Reflektors 21 kann variieren und insbesondere zwischen 100 und 1000 cm betragen. Insbesondere liegt der innere Durchmesser zwischen 200 bis 600 cm.

Nicht näher dargestellt ist, dass eine Auswerteeinrichtung zur Erfassung wenigstens einer chemischen und/oder physikalischen Größe vorgesehen sein kann. Insbesondere ist die Auswerteeinrichtung in dem ersten Verbindungsabschnitt 40 und/oder in der ersten Halteeinheit 23 angeordnet. Vorzugsweise weist die Auswerteeinrichtung einen Temperatursensor, einen UV-Sensor und/oder einen Geschwindigkeitssensor auf.

Ebenfalls nicht näher dargestellt ist, dass die Länge Z des ersten Haltemittels 25 und/oder des zweiten Haltemittels 35 zwischen $0,5 \cdot D_R$ bis $0,9 \cdot D_R$, bevorzugt zwischen $0,1 \cdot D_R$ bis $0,5 \cdot D_R$, beträgt, wobei $D_R$ den inneren Durchmesser des Reflektors 21, insbesondere den maximalen und/oder den mittleren inneren Durchmesser des Reflektors 21, bezeichnet.

**Ausführungsbeispiel:**

Bei durchgeführten Versuchen mit einer Bestrahlungsvorrichtung 1, wie sie in Fig. 9 dargestellt ist, hat sich gezeigt, dass mit einer Leistung von 140 W $\pm$ 10% bei einer Intensität an der Oberfläche der Strahlungsquellen 5 von etwa 4233 W/m$^2$ $\pm$ 10% bei verschiedenen Durchstromgeschwindigkeiten des Mediums deutlich verbesserte Abtötungsraten der Mikroorganismen erreicht werden können.

Dabei hat der Winkel $\alpha$ zwischen der Mittelachse S der jeweiligen Strahlungsquelle 5 und der Mittelachse R des Reflektors zwischen 2° bis 10°, insbesondere 2° $\pm$ 20%, betragen.

Das Gehäuse 4 hatte eine Länge von 100 cm bei einem Innendurchmesser von 15 cm. Die verwendeten Strahlungsquellen hatten einen Durchmesser von 5 cm +/-10 % bei einer Länge von 15 cm. Die verwendete Strahlungsquelle 5 hat UV-Strahlung bei einer Wellenlänge von 254 nm +/- 2 % bereitgestellt.

Bei den durchgeführten Versuchen konnte festgestellt werden, dass durch die schräge Anordnung der Strahlungsquellen 5 eine Erhöhung der UV-Strahlungsdosis erreicht werden kann - und zwar im Vergleich zu einem "gerade" ausgerichteten Lampenpaket. Unter einer geraden Ausrichtung ist zu verstehen, dass die Mittelachse S der Strahlungsquellen 5 zumindest im Wesentlichen parallel zur Mittelachse R des Reflektors verläuft.

Die Bestrahlungsdosis konnte in den durchgeführten Versuchen von etwa 600 J/m$^2$ bei der geraden Ausrichtung des Lampenpaketes auf wenigstens 800 bis wenigstens 850 J/m$^2$ erhöht werden.

Die vorgenannte UV-Strahlungsdosis ist biodosimetrisch - und insbesondere zusätzlich durch ein sogenanntes "internes Raytracing"-Verfahren - ermittelt worden und insbesondere auf Basis des View-Factors bestimmt worden. Letztlich ist die UV-Strahlungsdosis auf für den Fachmann auf bekannte Weise ermittelt worden. Im Hinblick auf die verwendeten Messmethoden darf auf die derzeit noch nicht fertiggestellte DIN(TS) 67506 (in Vorbereitung, Stand Juni 2021) und auf die ISO 15714 verwiesen werden.

Die Abtötungsrate der Mikroorganismen lag bei Viren ebenfalls über 99,99 % bei einem Volumenstrom von 700 m$^3$/h. Die Bakterien konnte darüber hinaus mit einer Abtötungsrate von 99 % abgetötet werden. Pilze konnten ferner mit einer Abtötungsrate von 50 % $\pm$ 3% abgetötet werden.

Als Medium ist mit Mikroorganismen belastete Luft gewählt worden.

Bei den durchgeführten Versuchen konnte festgestellt werden, dass durch die schräge Anordnung die konstruktive Interferenz auf vorteilhafte Weise zu einer Erhöhung der zu verabreichenden UV-Strahlungsdosis führen kann.

**Bezugszeichenliste:**

| | |
|---|---|
| 1 | Bestrahlungsvorrichtung |
| 2 | Einlass |
| 3 | Auslass |
| 4 | Gehäuse |
| 5 | Strahlungsquelle |
| 6 | Innenseite |
| 7 | Innenwandung |
| 8 | Behandlungskammer |
| 9 | Grund-Halteeinrichtung |
| 10 | Grund-Haltemittel |
| 11 | Längsachse von 4 |
| 12 | Längsachse von 5 |
| 13 | Länge von 4 |
| 14 | Durchmesser von 4 |
| 15 | Länge von 5 |
| 16 | Durchmesser von 5 |
| 17 | Ventilator |
| | |
| 21 | Reflektor |
| 22 | Halteeinrichtung |
| 23 | erste Halteeinheit |
| 24 | erstes Verbindungsmittel |
| 25 | erstes Haltemittel |
| 26 | Verbindungsbereich |
| 27 | stirnseitiger Endbereich von 5 |
| 28 | Endbereich von 25 |
| 29 | freier Endbereich von 25 |
| 30 | erstes Verstellmittel |
| 31 | Anordnungsbereich |
| 32 | Energieversorgungsleitung(en) |
| 33 | zweite Halteeinheit |
| 34 | zweites Verbindungsmittel |
| 35 | zweites Haltemittel |
| 36 | zweiter Verbindungsbereich |
| 37 | weiterer stirnseitiger Endbereich von 5 |
| 38 | Endbereich von 35 |
| 39 | freier Endbereich von 35 |
| 40 | erster Verbindungsabschnitt |
| 41 | erste Versorgungseinrichtung |
| 42 | Vorschaltgeräte |
| 43 | zweiter Verbindungsabschnitt |
| 44 | weiterer Verbindungsabschnitt |
| 45 | Verbindungsteil |
| 46 | Haltestreben |
| 47 | Befestigungsmittel |
| | |
| $\alpha$ | Winkel |
| $\beta$ | Winkel zwischen 25 |
| $\gamma$ | Winkel zwischen 25 |
| $\delta$ | Winkel zwischen 5 |
| | |
| $S, S_1, S_2$ | Mittelachse Strahlungsquelle |
| R | Mittelachse Reflektor |
| D | Durchmesser von 5 |
| L | Länge von 5 |
| $Z, Z_1, Z_2$ | Länge erstes bzw. zweites Haltemittel |

**Patentansprüche**

1. Bestrahlungsvorrichtung (1) zur UV-Bestrahlung, insbesondere UV-C-Bestrahlung, eines die Bestrahlungsvorrichtung (1) durchströmenden gasförmigen Mediums, insbesondere Luft, insbesondere zur Inaktivierung von in dem Medium befindlichen Mikroorganismen, wie Bakterien, Keime, Schimmel und/oder Viren, mit einem einen Einlass (2) und einen Auslass (3) für das Medium aufweisenden zu durchströmenden Gehäuse (4) und wenigstens einer im Inneren des Gehäuses (4) angeordneten, UV-Strahlung emittierenden Strahlungsquelle (5) zur Bestrahlung des das Gehäuse (4) durchströmenden Mediums,

   wobei das Gehäuse (4) einen Reflektor (21) aufweist, wobei der Reflektor (21) an der der Strahlungsquelle (5) zugewandten Innenseite (6) zumindest bereichsweise, vorzugsweise vollflächig, reflektierend mit einem Reflexionsgrad für die von der Strahlungsquelle (5) emittierte UV-Strahlung von wenigstens 0,6 ausgebildet ist,
   wobei die wenigstens eine Strahlungsquelle (5) mittels einer Halteeinrichtung (22) gehalten und/oder fixiert ist, wobei die Halteeinrichtung (22), vorzugsweise lösbar, mit dem Gehäuse (4) verbunden ist, wobei die Halteeinrichtung (22) derart ausgebildet ist, dass die Mittelachse (S) der wenigstens einen Strahlungsquelle (5) einen Winkel ($\alpha$) zu der Mittelachse des Reflektors (R) einschließt,
   wobei eine Mehrzahl von Strahlungsquellen (5) an der Halteeinrichtung (22) gehalten und/oder fixiert ist,
   wobei die Halteeinrichtung (22) eine erste Halteeinheit (23) aufweist, wobei die erste Halteeinheit (23) über ein erstes Verbindungsmittel (24) der Halteeinrichtung (22) mit dem Gehäuse (4) lösbar verbindbar ist,
   wobei die erste Halteeinheit (23) eine Mehrzahl von zueinander zumindest bereichsweise beabstandeten ersten Haltemitteln (25) aufweist,
   wobei die ersten Haltemittel (25) als, insbesondere stegförmige, Haltearme ausgebildet sind,
   wobei die ersten Haltemittel (25) mit einem Verbindungsbereich (26) der ersten Halteeinheit (23) verbunden sind, wobei der Verbindungsbereich (26) mit dem Verbindungsmittel (24) verbunden ist und/oder einstückig mit diesem ausgebildet ist,
   wobei die Strahlungsquelle (5) an einem stirnseitigen Endbereich (27) mit dem ersten Haltemittel (25), vorzugsweise lösbar und formschlüssig, kraftschlüssig und/oder reibschlüssig, verbunden ist, wobei das erste Haltemittel (25) mit einem Endbereich (28) mit dem Verbindungsbereich (26) verbunden ist,

wobei das erste Haltemittel (25) an seinem freien Endbereich (29) mit der Strahlungsquelle (5) verbunden ist,

wobei eine zweite Halteeinheit (33) vorgesehen ist, wobei die zweite Halteeinheit (33) über wenigstens ein zweites Verbindungsmittel (34) der Halteeinrichtung (22) mit dem Gehäuse (4), vorzugsweise lösbar, verbindbar ist, wobei die zweite Halteeinheit (33) eine Mehrzahl von zueinander zumindest bereichsweise beabstandeten zweiten Haltemitteln (35), vorzugsweise ausgebildet als, insbesondere stegförmige, Haltearme, aufweist und

wobei die Strahlungsquelle (5) an einem weiteren stirnseitigen Endbereich (37) mit dem zweiten Haltemittel (35), vorzugsweise lösbar und formschlüssig, kraftschlüssig und/oder reibschlüssig, verbunden ist .

2. Bestrahlungsvorrichtung nach einem der vorhergehenden Ansprüche, **dadurch gekennzeichnet, dass** jede Mittelachse (S, S₁, S₂) jeder Strahlungsquelle (5) einen Winkel ($\alpha$) zu der Mittelachse (R) des Reflektors (21) einschließt.

3. Bestrahlungsvorrichtung nach einem der vorhergehenden Ansprüche, **dadurch gekennzeichnet, dass** die Strahlungsquelle (5) lösbar mit der Halteinrichtung (22) verbunden ist.

4. Bestrahlungsvorrichtung nach einem der vorhergehenden Ansprüche, **dadurch gekennzeichnet, dass** wenigstens ein erstes Haltemittel (25) über ein mit dem Verbindungsbereich (26) verbundenes erstes Verstellmittel (24) verstellbar ist, insbesondere derart, die Schrägstellung der Mittelachse (S) der an dem ersten Haltemittel (25) befestigten Strahlungsquelle (5) in Bezug zu der Mittelachse (R) des Reflektors (21) verstellbar ist und/oder

dass die ersten Haltemittel (25) langgestreckt ausgebildet sind und dass wenigstens zwei erste Haltemittel (25) eine sich voneinander unterscheidende Länge (Z) aufweisen und/oder dass wenigstens ein erstes Haltemittel (25) wenigstens zwei Anordnungsbereiche (31) zur Verbindung mit der Strahlungsquelle (5) aufweist und/oder

dass die erste Halteeinheit (23) derart ausgebildet ist, dass wenigstens zwei zwischen zwei unmittelbar benachbarten ersten Haltemitteln (25) eingeschlossene Winkel ($\beta$, $\gamma$) zueinander unterschiedlich ausgebildet sind, insbesondere um wenigstens 5%, bevorzugt wenigstens 10%, zueinander abweichen.

5. Bestrahlungsvorrichtung nach einem der vorhergehenden Ansprüche, **dadurch gekennzeichnet, dass** die erste Halteeinheit (23) zur Energiezuführung zu den Strahlungsquellen (5) ausgebildet ist, insbesondere wobei in dem ersten Verbindungsmittel (24), in der ersten Halteeinheit (23) und/oder in den ersten Haltemitteln (25) Energieversorgungsleitungen (32) angeordnet .

6. Bestrahlungsvorrichtung nach einem der vorhergehenden Ansprüche, **dadurch gekennzeichnet, dass** die erste Halteeinheit (23), insbesondere der Verbindungsbereich (26), mit der zweiten Halteeinheit (33), insbesondere mit einem zweiten Verbindungsbereich (36), der insbesondere die zweiten Haltemittel (35) mit dem zweiten Verbindungsmittel (34) verbindet, über ein langgestrecktes, vorzugsweise steifes, Verbindungsteil (45) verbunden ist und/oder

dass die Strahlungsquelle (5) und/oder die Strahlungsquellen (5) beidseitig an der ersten und der zweiten Halteeinheit (23, 33), insbesondere jeweils in einem stirnseitigen Endbereich (27, 37), gelagert ist/sind und/oder dass die zweite Halteeinheit (33) zumindest im Wesentlichen baugleich zur ersten Halteeinheit (23) ausgebildet ist.

7. Bestrahlungsvorrichtung nach einem der vorhergehenden Ansprüche, **dadurch gekennzeichnet, dass** die erste Halteeinheit (23) und/oder das erste Verbindungsmittel (24) mit einem ersten Verbindungsabschnitt (40) verbunden ist/sind, insbesondere wobei der erste Verbindungsabschnitt (40) mit dem Gehäuse (4) und/oder dem Reflektor (21) lösbar verbindbar ist und/oder insbesondere wobei der erste Verbindungsabschnitt (40) zumindest teilweise über das Gehäuse (4) absteht und/oder insbesondere wobei an dem ersten Verbindungsabschnitt (40) außenseitig eine erste Versorgungseinrichtung (41), vorzugsweise aufweisend eine Mehrzahl von Vorschaltgeräten (42), anordnenbar und/oder verbindbar ist, wobei, vorzugsweise, die erste Versorgungseinrichtung (41) elektrisch über den ersten Verbindungsabschnitt (40) mit der ersten Halteeinheit (23) verbunden ist und/oder dass die zweite Halteeinheit (33) und/oder das zweite Verbindungsmittel (34) mit einem zweiten Verbindungsabschnitt (43) verbunden ist/sind, insbesondere wobei der zweite Verbindungsabschnitt (43) mit dem Gehäuse (4) und/oder dem Reflektor (21) lösbar verbindbar ist und/oder insbesondere wobei der zweite Verbindungsabschnitt (43) zumindest teilweise über das Gehäuse (4) absteht.

8. Bestrahlungsvorrichtung nach Anspruch 7, **dadurch**

**gekennzeichnet, dass** der erste und der zweite Verbindungsabschnitt (40, 43) lösbar miteinander formschlüssig und/oder reibschlüssig und/oder kraftschlüssig verbindbar und/oder verbunden sind und/oder.

dass wenigstens ein weiterer Verbindungsabschnitt (44) vorgesehen ist, der lösbar mit dem ersten und/oder zweiten Verbindungsabschnitt (40, 43) formschlüssig und/oder reibschlüssig und/oder kraftschlüssig verbindbar und/oder verbunden ist und/oder

dass der erste, zweite und/oder weitere Verbindungsabschnitt (40, 43, 44) zumindest teilweise in das Innere des Reflektors (21) hineinragt und/oder an die Innenseite (6) des Reflektors (21) angrenzt.

9. Bestrahlungsvorrichtung nach einem der vorhergehenden Ansprüche, **dadurch gekennzeichnet, dass** zwischen 3 bis 25, bevorzugt zwischen 4 bis 15, weiter bevorzugt zwischen 5 bis 10, Strahlungsquellen (5), erste Haltemittel (25) und/oder zweite Haltemittel (35) vorgesehen sind.

10. Bestrahlungsvorrichtung nach einem der vorhergehenden Ansprüche, **dadurch gekennzeichnet, dass** die Strahlungsquellen (5) baugleich ausgebildet sind.

11. Bestrahlungsvorrichtung nach einem der vorhergehenden Ansprüche, **dadurch gekennzeichnet, dass** wenigstens eine, bevorzugt alle, Strahlungsquelle (5) einen Durchmesser (D) zwischen 1 cm bis 20 cm, bevorzugt zwischen 2 bis 10 cm und weiter bevorzugt zwischen 4 bis 6 cm, aufweist.

12. Bestrahlungsvorrichtung nach einem der vorhergehenden Ansprüche, **dadurch gekennzeichnet, dass** wenigstens eine, bevorzugt alle, Strahlungsquelle (5) eine Länge (L) zwischen 0,2 m bis 10 m, bevorzugt zwischen 0,5 bis 5 m, weiter bevorzugt zwischen 1 bis 2 m, aufweist.

**Claims**

1. Irradiation device (1) for UV irradiation, in particular UV-C irradiation, of a gaseous medium flowing through the irradiation device (1), in particular air, in particular for inactivating microorganisms present in the medium, such as bacteria, germs, mold and/or viruses, with a housing (4) through which the medium is to flow, having an inlet (2) and an outlet (3), and at least one radiation source (5), which is arranged in the interior of the housing (4) and emits UV radiation, for irradiating the medium flowing through the housing (4),

wherein the housing (4) has a reflector (21), wherein the reflector (21) on the inner side (6) facing the radiation source (5) is at least partially, preferably fully, reflective with a degree of reflection for the UV radiation emitted by the radiation source (5) of at least 0.6,

wherein the at least one radiation source (5) is held and/or fixed by means of a holding device (22), wherein the holding device (22) is connected, preferably detachably, to the housing (4), wherein the holding device (22) is designed in such a way that the central axis (S) of the at least one radiation source (5) encloses an angle ($\alpha$) to the central axis of the reflector (R),

wherein a plurality of radiation sources (5) is held and/or fixed to the holding device (22),

wherein the holding device (22) has a first holding unit (23), the first holding unit (23) being releasably connectable to the housing (4) via a first connecting means (24) of the holding device (22),

wherein the first holding unit (23) has a plurality of first holding means (25) spaced apart from one another at least in regions,

wherein the first holding means (25) are designed as, in particular web-shaped, holding arms,

wherein the first holding means (25) are connected to a connection area (26) of the first holding unit (23), wherein the connection area (26) is connected to the connecting means (24) and/or is formed integrally therewith,

wherein the radiation source (5) is connected at a front end area (27) to the first holding means (25), preferably releasably and positively, non-positively and/or frictionally, wherein the first holding means (25) is connected with an end area (28) to the connection area (26),

wherein the first holding means (25) is connected at its free end area (29) to the radiation source (5),

wherein a second holding unit (33) is provided, the second holding unit (33) being connectable, preferably detachably, to the housing (4) via at least one second connecting means (34) of the holding device (22), the second holding unit (33) having a plurality of second holding means (35) spaced apart from one another at least in regions, preferably designed as, in particular web-shaped, holding arms and

wherein the radiation source (5) is connected at a further front end area (37) to the second holding means (35), preferably releasably and positively, non-positively and/or frictionally.

2. Irradiation device according to one of the preceding claims, **characterized in that** each central axis (S, $S_1$, $S_2$) of each radiation source (5) includes an angle

($\alpha$) to the central axis (R) of the reflector (21).

3. Irradiation device according to one of the preceding claims, **characterized in that** the radiation source (5) is detachably connected to the holding device (22).

4. Irradiation device according to one of the preceding claims, **characterized in that** at least a first holding means (25) is adjustable via a first adjusting means (24) connected to the connection area (26), in particular in such a way that the oblique position of the central axis (S) of the radiation source (5) fastened to the first holding means (25) is adjustable with respect to the central axis (R) of the reflector (21) and/or

   **in that** the first holding means (25) are elongated and **in that** at least two first holding means (25) have a length (Z) differing from one another and/or **in that** at least one first holding means (25) has at least two arrangement areas (31) for connection to the radiation source (5) and/or **in that** the first holding unit (23) is designed in such a way that at least two angles ($\beta$, $\gamma$) enclosed between two directly adjacent first holding means (25) are designed to differ from one another, in particular by at least 5%, preferably at least 10%.

5. Irradiation device according to one of the preceding claims, **characterized in that** the first holding unit (23) is designed for supplying energy to the radiation sources (5), in particular wherein energy supply lines (32) are arranged in the first connecting means (24), in the first holding unit (23) and/or in the first holding means (25).

6. Irradiation device according to one of the preceding claims, **characterized in that** the first holding unit (23), in particular the connection area (26), is connected to the second holding unit (33), in particular to a second connection area (36), which in particular connects the second holding means (35) to the second connecting means (34), via an elongated, preferably rigid, connecting part (45) and/or

   **in that** the radiation source (5) and/or the radiation sources (5) is/are mounted on both sides of the first and the second holding unit (23, 33), in particular in each case in a front end area (27, 37) and/or **in that** the second holding unit (33) is at least substantially identical in construction to the first holding unit (23).

7. Irradiation device according to one of the preceding claims, **characterized in that** the first holding unit (23) and/or the first connecting means (24) is/are connected to a first connection section (40), in particular wherein the first connection section (40) can be detachably connected to the housing (4) and/or the reflector (21) and/or in particular wherein the first connection section (40) projects at least partially beyond the housing (4) and/or in particular wherein a first supply device (41) can be arranged and/or connected on the outside of the first connection section (40), preferably comprising a plurality of ballasts (42), can be arranged and/or connected on the outside of the first connection section (40), wherein, preferably, the first supply device (41) is electrically connected to the first holding unit (23) via the first connection section (40) and/or

   **in that** the second holding unit (33) and/or the second connecting means (34) is/are connected to a second connection section (43), in particular wherein the second connection section (43) is detachably connectable to the housing (4) and/or the reflector (21) and/or in particular wherein the second connection section (43) protrudes at least partially beyond the housing (4).

8. Irradiation device according to claim 7, **characterized in that** the first and the second connection sections (40, 43) are detachably connectable and/or connected to each other in a form-fitting and/or friction-fitting and/or force-fitting manner and/or

   **in that** at least one further connection section (44) is provided, which is detachably connectable and/or connected to the first and/or second connection section (40, 43) in a form-fitting and/or friction-fitting and/or force-fitting manner and/or **in that** the first, second and/or further connection sections (40, 43, 44) projects at least partially into the interior of the reflector (21) and/or adjoins the inner side (6) of the reflector (21).

9. Irradiation device according to one of the preceding claims, **characterized in that** between 3 to 25, preferably between 4 to 15, more preferably between 5 to 10, radiation sources (5), first holding means (25) and/or second holding means (35) are provided.

10. Irradiation device according to one of the preceding claims, **characterized in that** the radiation sources (5) are of identical construction.

11. Irradiation device according to any one of the preceding claims, **characterized in that** at least one, preferably all, radiation sources (5) has a diameter (D) between 1 cm to 20 cm, preferably between 2 to 10 cm and more preferably between 4 to 6 cm.

12. Irradiation device according to one of the preceding claims, **characterized in that** at least one, preferably all, radiation source (5) has a length (L) between 0.2 m to 10 m, preferably between 0.5 to 5 m, more preferably between 1 to 2 m.

## Revendications

1. Dispositif d'irradiation (1) pour l'irradiation UV, en particulier l'irradiation UV-C, d'un médium gazeux traversant le dispositif d'irradiation (1), en particulier de l'air, en particulier pour l'inactivation de micro-organismes se trouvant dans le médium, tels que des bactéries, des germes, des moisissures et/ou des virus, avec un boîtier (4) à traverser, présentant une entrée (2) et une sortie (3) pour le médium, et au moins une source de rayonnement (5) disposée à l'intérieur du boîtier (4) et émettant un rayonnement UV pour l'irradiation du médium traversant le boîtier (4),

   le boîtier (4) présentant un réflecteur (21), le réflecteur (21) étant réalisé de manière à être réfléchissant au moins par zones, de préférence sur toute sa surface, sur le côté intérieur (6) tourné vers la source de rayonnement (5), avec un degré de réflexion pour le rayonnement UV émis par la source de rayonnement (5) d'au moins 0,6,
   dans lequel la au moins une source de rayonnement (5) est maintenue et/ou fixée au moyen d'un dispositif de maintien (22), dans lequel le dispositif de maintien (22) est relié, de préférence de manière amovible, au boîtier (4), dans lequel le dispositif de maintien (22) est conçu de telle sorte que l'axe central (S) de la au moins une source de rayonnement (5) forme un angle (α) avec l'axe central du réflecteur (R),
   dans lequel une pluralité de sources de rayonnement (5) est maintenue et/ou fixée sur le dispositif de maintien (22),
   dans lequel le dispositif de maintien (22) comprend une première unité de retenue (23), la première unité de maintien (23) pouvant être reliée de manière amovible au boîtier (4) par un premier moyen de liaison (24) du dispositif de maintien (22),
   la première unité de maintien (23) présentant une pluralité de premiers moyens de maintien (25) espacés les uns des autres au moins par zones,
   les premiers moyens de maintien (25) étant conçus comme des bras de retenue, en particulier en forme de barrette,
   dans lequel les premiers moyens de maintien (25) sont reliés à une partie de liaison (26) de la première unité de maintien (23), la partie de liaison (26) étant reliée aux moyens de liaison (24) et/ou étant formée d'un seul tenant avec ceux-ci,
   la source de rayonnement (5) étant reliée à une zone d'extrémité (27) frontale au premier moyen de maintien (25), de préférence de manière amovible et par complémentarité de forme, par adhérence et/ou par friction, le premier moyen de maintien (25) étant relié par une zone d'extrémité (28) à la zone de liaison (26),
   dans lequel le premier moyen de maintien (25) est relié à la source de rayonnement (5) au niveau de sa partie d'extrémité libre (29),
   une deuxième unité de maintien (33) prévue, la deuxième unité de maintien (33) pouvant être reliée, de préférence de manière amovible, au boîtier (4) par l'intermédiaire d'au moins un deuxième moyen de liaison (34) du dispositif de maintien (22), la deuxième unité de maintien (33) présentant une pluralité de deuxièmes moyens de maintien (35) espacés les uns des autres au moins par zones, de préférence conçus comme des bras de maintien, en particulier en forme de barrette, et
   la source de rayonnement (5) étant reliée au niveau d'une autre zone d'extrémité frontale (37) au deuxième moyen de maintien (35), de préférence de manière amovible et par complémentarité de forme, par adhérence et/ou par friction.

2. Dispositif d'irradiation selon l'une des revendications précédentes, **caractérisé en ce que** chaque axe central (S, $S_1$, $S_2$) de chaque source de rayonnement (5) forme un angle (α) avec l'axe central (R) du réflecteur (21).

3. Dispositif d'irradiation selon l'une des revendications précédentes, **caractérisé en ce que** la source de rayonnement (5) est reliée de manière amovible au dispositif de maintien (22).

4. Dispositif d'irradiation selon l'une quelconque des revendications précédentes, **caractérisé en ce qu'**au moins un premier moyen de maintien (25) est réglable par l'intermédiaire d'un premier moyen de réglage (24) relié à la zone de liaison (26), en particulier de manière à régler l'inclinaison de l'axe central (S) de la source de rayonnement (5) fixée sur le premier moyen de maintien (25) par rapport à l'axe central (R) du réflecteur (21), et/ou

   **en ce que** les premiers moyens de maintien (25) sont réalisés de manière allongée et **en ce qu'**au moins deux premiers moyens de maintien (25) présentent une longueur (Z) différente l'une de l'autre et/ou **en ce qu'**au moins un premier moyen de maintien (25) présente au moins deux zones d'agencement (31) pour la liaison avec la source de rayonnement (5) et/ou

**en ce que** la première unité de maintien (23) est réalisée de telle sorte qu'au moins deux angles (β, γ) inclus entre deux premiers moyens de maintien (25) directement voisins sont réalisés différemment l'un de l'autre, en particulier différent l'un de l'autre d'au moins 5%, de préférence d'au moins 10%.

5. Dispositif d'irradiation selon l'une des revendications précédentes, **caractérisé en ce que** la première unité de maintien (23) est conçue pour l'alimentation en énergie des sources de rayonnement (5), en particulier dans lequel des lignes d'alimentation en énergie (32) sont disposées dans le premier moyen de liaison (24), dans la première unité de maintien (23) et/ou dans les premiers moyens de maintien (25).

6. Dispositif d'irradiation selon l'une des revendications précédentes, **caractérisé en ce que** la première unité de maintien (23), en particulier la zone de liaison (26), est reliée à la deuxième unité de maintien (33), en particulier à une deuxième zone de liaison (36) reliant en particulier les deuxièmes moyens de maintien (35) au deuxième moyen de liaison (34), par l'intermédiaire d'une pièce de liaison (45) allongée, de préférence rigide, et/ou

   **en ce que** la source de rayonnement (5) et/ou les sources de rayonnement (5) est/sont montée(s) des deux côtés sur la première et la deuxième unité de maintien (23, 33), en particulier respectivement dans une zone d'extrémité frontale (27, 37) et/ou
   **en ce que** la deuxième unité de maintien (33) est réalisée au moins essentiellement de manière identique à la première unité de maintien (23).

7. Dispositif d'irradiation selon l'une des revendications précédentes, **caractérisé en ce que** la première unité de maintien (23) et/ou le premier moyen de liaison (24) est/sont relié(s) à une première section de liaison (40), en particulier la première section de liaison (40) pouvant être reliée de manière amovible au boîtier (4) et/ou au réflecteur (21) et/ou en particulier la première section de liaison (40) dépassant au moins partiellement du boîtier (4) et/ou en particulier un premier dispositif d'alimentation (41) étant monté sur le côté extérieur de la première section de liaison (40), présentant de préférence une pluralité de ballasts (42), le premier dispositif d'alimentation (41) étant de préférence relié électriquement à la première unité de maintien (23) par l'intermédiaire de la première section de liaison (40), et/ou
   **en ce que** la deuxième unité de maintien (33) et/ou le deuxième moyen de liaison (34) est/sont relié(s) à une deuxième section de liaison (43), en particulier la deuxième section de liaison (43) pouvant être relié de manière amovible au boîtier (4) et/ou au réflecteur (21) et/ou en particulier la deuxième section de liaison (43) dépassant au moins partiellement du boîtier (4).

8. Dispositif d'irradiation selon la revendication 7, **caractérisé en ce que** la première et la deuxième section de liaison (40, 43) peuvent être reliées et/ou sont reliées l'une à l'autre de manière détachable par engagement positif et/ou par friction et/ou par adhérence et/ou

   **en ce qu'**il est prévu au moins une autre section de liaison (44) qui peut être reliée et/ou est reliée de manière amovible à la première et/ou à la deuxième section de liaison (40, 43) par complémentarité de forme et/ou par friction et/ou par adhérence et/ou
   **en ce que** le premier, la deuxième et/ou l'autre section de liaison (40, 43, 44) pénètrent au moins partiellement à l'intérieur du réflecteur (21) et/ou sont adjacents à la face intérieure (6) du réflecteur (21).

9. Dispositif d'irradiation selon l'une quelconque des revendications précédentes, **caractérisé en ce qu'**il est prévu entre 3 et 25, de préférence entre 4 et 15, plus préférentiellement entre 5 et 10, sources de rayonnement (5), premiers moyens de maintien (25) et/ou deuxièmes moyens de maintien (35).

10. Dispositif d'irradiation selon l'une des revendications précédentes, **caractérisé en ce que** les sources de rayonnement (5) sont de construction identique.

11. Dispositif d'irradiation selon l'une quelconque des revendications précédentes, **caractérisé en ce qu'**au moins une source de rayonnement (5), de préférence toutes, présente un diamètre (D) compris entre 1 cm et 20 cm, de préférence entre 2 et 10 cm, et plus préférentiellement entre 4 et 6 cm.

12. Dispositif d'irradiation selon l'une quelconque des revendications précédentes, **caractérisé en ce qu'**au moins une source de rayonnement (5), de préférence toutes les sources de rayonnement, présente une longueur (L) comprise entre 0,2 m et 10 m, de préférence entre 0,5 et 5 m, plus préférentiellement entre 1 et 2 m.

Fig. 1

Fig. 2

Fig. 3

Fig. 4

Fig. 5

Fig. 6

Fig. 7

Fig. 8

EP 4 168 056 B1

Fig. 9

Fig. 10

Fig. 11

Fig. 12

Fig. 13

**Fig. 14**

**Fig. 15**

Fig. 16

Fig. 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20190491239 A1
- DE 102017117324 A1
- DE 20021236 U1
- EP 3111963 A1
- KR 20170124845 A
- WO 9307091 A1
- RU 2417105 C1
- US 2005092932 A1